(19) **Europäisches Patentamt** **European Patent Office** **Office européen des brevets**

(11) **EP 3 298 996 B1**

(12) **EUROPEAN PATENT SPECIFICATION**

(45) Date of publication and mention of the grant of the patent:
**26.04.2023 Bulletin 2023/17**

(21) Application number: **16796172.1**

(22) Date of filing: **17.03.2016**

(51) International Patent Classification (IPC):
**A61F 13/02** (2006.01)

(52) Cooperative Patent Classification (CPC):
**A61F 13/0236; A61F 13/0243; A61F 13/0253**

(86) International application number:
**PCT/JP2016/058518**

(87) International publication number:
**WO 2016/185778 (24.11.2016 Gazette 2016/47)**

(54) **ADHESIVE SKIN PATCH MATERIAL, AND SUPPORT FOR ADHESIVE SKIN PATCH MATERIAL WHICH CAN BE USED IN SAME**

HAUTPFLASTERMATERIAL UND TRÄGER FÜR HAUTPFLASTERMATERIAL ZUR VERWENDUNG DAFÜR

MATÉRIAU DE TIMBRE TRANSDERMIQUE ADHÉSIF ET SUPPORT POUR MATÉRIAU DE TIMBRE TRANSDERMIQUE ADHÉSIF POUVANT ÊTRE UTILISÉ DANS CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.05.2015 JP 2015103194**

(43) Date of publication of application:
**28.03.2018 Bulletin 2018/13**

(73) Proprietors:
• **Nichiban Company Limited**
  **Bunkyo-ku,**
  **Tokyo 112-8663 (JP)**
• **KB Seiren, Ltd.**
  **Sabae-shi**
  **Fukui 916-0038 (JP)**

(72) Inventors:
• **IKAI, Tomonori**
  **Tokyo 112-8663 (JP)**
• **FUJISAWA, Hiromichi**
  **Tokyo 112-8663 (JP)**
• **OTAKE, Kosuke**
  **Osaka-shi**
  **Osaka 530-0001 (JP)**
• **MIYAHARA, Kazuhisa**
  **Nagahama-shi**
  **Shiga 526-0068 (JP)**

(74) Representative: **Mewburn Ellis LLP**
  **Aurora Building**
  **Counterslip**
  **Bristol BS1 6BX (GB)**

(56) References cited:
WO-A2-2008/019051   JP-A- H11 200 124
JP-A- S59 183 751   JP-A- 2006 207 092
JP-A- 2009 545 382   JP-A- 2010 281 004
JP-A- 2012 135 582   JP-A- 2012 135 582
JP-A- 2013 053 386

**Description**

TECHNICAL FIELD

The present invention relates to an adhesive skin patch, particularly to an adhesive skin patch for covering wound or for protecting post-surgical wound, further an adhesive skin patch for healing wounds, for treating scars or for preventing formation of scars and keloids, and a support layer for an adhesive skin patch to be used for the same.

BACKGROUND ART

Wounds made on the skin by external wound or surgery generally cure in about two weeks, and will continue to change over the about six months thereafter. In general, scars become stiff and reddish after about 1 month, but they gradually become soft over the next several months and become the same color as the surrounding skin. However, if the process of normal wound healing is impaired by a certain reason, there are some cases where the wound does not heal and becomes chronic, or causes abnormal way of healing. One of abnormal methods of healing is called scars or keloids. Although the mechanism by which scars or keloids arise is unknown, it has also been pointed out that it relates to the mechanical tension applied onto the skin and the strength of inflammation.
Conventionally, as a method for treating scars or preventing formation of scars or keloids, a method of reducing external forces applied to the skin due to an external wound or post-surgery has been used (see Patent documents 1 to 3). Specifically, Patent document 1 discloses a hydrocolloid adhesive skin patch having a specific elongation force. Patent document 2 discloses a device, a bandage, a kit and a method that is configured to shield a wound from endogenous stress and/or external stress, which can improve formation of scars and/or keloid. In addition, Patent document 3 discloses a material for treating skin disorder comprising an adhesive skin patch which is applied corresponding to a peripheral zone adjacent to an affected part such as keloids, etc., an elastic body which covers the affected part, and an adhesive skin patch for supporting the elastic body so as to at least partially cover the above-mentioned adhesive skin patch while supporting the elastic body from above and pressing the above-mentioned elastic body toward the affected part.

PRIOR ART DOCUMENTS

Patent Documents

Patent document 1: JP 2012-135582A
Patent document 2: JP 2009-545382A
Patent document 3: JP Hei. 07-079827B

JP 2010 281004 A describes a woven fabric prepared by using a copolymerized polyester multi-filament (1) as a warp and a hot-fusible filament (2) at least as a part and keeps the copolymerized polyester multi-filament and the hot-fusible filament fused and fixed at their crossing parts. (1) The copolymerized polyester multi-filament consists of the polyester in which the main repeating unit is ethylene terephthalate, and which incorporates 2.0-3.0 mol% metal sulfonate group-containing isophthalic acid component to the acid components of the whole polyester and contains a polyalkylene glycol with a mean molecular weight of 150-600. (2) The hot-fusible filament comprises a hot-fusible component with a softening point of 120-220°C on the fiber surface.

SUMMARY OF THE INVENTION

Problems to be solved by the Invention

However, in the conventional hydrocolloid adhesive skin patch, a bandage or a material for treating skin disorder, etc., there are disadvantages that whereas it suppresses elongation of the skin, the support layer does not stretch so that followability to the skin is low and it cannot be adhered for a long time. Therefore, it has been desired to provide an adhesive skin patch for preventing formation of scars and keloids which is capable of suppressing elongation of the skin, as well as excellent in followability to the skin and is capable of being adhered for a long time. An object of the present invention is to provide an adhesive skin patch for covering wound or for protecting post-surgical wound which can suppress elongation of the skin, as well as excellent in followability to the skin and is capable of being adhered for a long time, in particular, an adhesive skin patch for healing wounds, for treating scars or for preventing formation of scars and keloids. In addition, another object of the present invention is to provide a support layer for an adhesive skin patch which is suitable for use as the above-mentioned adhesive skin patch.

Means to Solve the Problems

The invention which has been made to solve the above-mentioned problems is an adhesive skin patch for covering wound or for protecting post-surgical wound as set out in the appended claim 1. The adhesive skin patch of the present invention can suppress elongation of the skin, is further excellent in followability to the skin, and is capable of being adhered for a long time. The adhesive skin patch of the present invention is also excellent in long term adhesion which has never been provided in the conventional adhesive skin patch sufficiently. The present inventors have found that when a 1% tensile load of the adhesive skin patch is 13 N/25 mm or less and a 10% tensile load of the same is 18 N/25 mm or more and 95 N/25 mm or less, such an adhesive skin patch can appropriately and sufficiently suppress elongation of the skin in response to the stress to stretch the skin, is also excellent in followability to the skin, and is capable of being adhered for a long time. That is, in the adhesive skin patch of the present invention, the load at low elongation is low, and the load at elongation to a certain extent is high. According to this property, the adhesive skin patch of the present invention has the characteristic that it stretches to sufficiently follow the skin against a weak stress that does not greatly affect the wound part, and firmly holds against a strong stress that exerts an influence on the wound part whereby it has effects of healing wounds, treating scars or preventing formation of scars or keloids.

That is, the gist of the present invention is as follows. In the present invention, the "apparent density $(g/cm^3)$" means a weight (g) per a unit volume of the adhesive skin patch represented by the number of grams per cubic meter, and is a value in which a basis weight $(g/m^2)$ is divided by a thickness $(\mu m)$.

[1] An adhesive skin patch for covering wound or for protecting post-surgical wound which comprises a support layer and a pressure-sensitive adhesive layer provided on one side of the support layer, wherein a 1% tensile load in a machine direction and a cross machine direction of the adhesive skin patch is 13 N/25 mm or less and a 10% tensile load of the same is 18 N/25 mm or more and 95 N/25 mm or less, wherein the machine direction is the direction along the long side of the adhesive skin patch and the cross machine direction is the direction along the short side, wherein the tensile load is measured using JIS K7113, and wherein the support layer comprises a woven fabric having a warp density of 80 to 120 yarns/inch (2.54 cm) and a weft density of 80 to 120 yarns/inch (2.54 cm), and using yarns having a total fineness of 40 dtex or more and 85 dtex or less, wherein a moisture permeability $(g/m^2 \cdot 24h)$ of the adhesive skin patch is 1,000 $g/m^2 \cdot 24h$ or more, wherein the moisture permeability is measured according to condition B of JIS Z 0208, at a temperature of 40°C and a relative humidity of 90%..

[2] The adhesive skin patch described in [1], wherein a bending resistance (mm) of the adhesive skin patch according to JIS L1096 is 5 mm or more and 40 mm or less.

[3] The adhesive skin patch described in [1] or [2], wherein a 5% tensile load in the machine direction and the cross machine direction of the adhesive skin patch is 5 N/25 mm or more and 50 N/25 mm or less.

[4] The adhesive skin patch described in any one of [1] to [3], wherein a product of the bending resistance (mm) according to the cantilever method and an apparent density $(g/cm^3)$ is 2 or more and 30 or less

[5] The adhesive skin patch described in any one of [1] to [4], wherein an elongation recovery rate of at least one of the machine direction and the cross machine direction of the adhesive skin patch at a load of 9 N and 18 N is 85% or more.

[6] The adhesive skin patch described in any one of [1] to [5], for use in healing wounds, for treating scars or for preventing formation of scars and keloids.

[7] The adhesive skin patch described in [6], for use in healing wounds of a wound by surgery, for treating scars or for preventing formation of scars and keloids.

[8] The adhesive skin patch described in [7] wherein the surgery is a caesarean section, a median sternotomy incision or a laparoscopic operation.

[9] An adhesive skin patch according to [1], for use in healing wounds of a wound by surgery, treating scars or preventing formation of scars and keloids, wherein a 5% tensile load of the same is 5 N/25 mm or more and 50 N/25 mm or less, a 10% tensile load of the same is 18 N/25 mm or more and 95 N/25 mm or less, and the pressure-sensitive adhesive layer contains a foamed acrylic-based adhesive.

[10] An adhesive skin patch according to [1] for use in healing wounds of a wound by surgery, treating scars or preventing formation of scars and keloids, wherein

a 5% tensile load in a machine direction and a cross machine direction of the adhesive skin patch is 5 N/25 mm or more and 50 N/25 mm or less, a bending resistance (mm) of the adhesive skin patch is 8 mm or more and 25 mm or less, and a shape of which has one or more concave portions or convex portions on a side portion sandwiched between both ends in a machine direction or a cross machine direction.

[11] A support layer for an adhesive skin patch which is used for a support layer of an adhesive skin patch for covering wound or for protecting post-surgical wound, wherein a 1% tensile load in a machine direction and a cross machine direction of the support layer is 13 N/25 mm or less and a 10% tensile load of the same is 15 N/25 mm or more and 95 N/25 mm or less, wherein the machine direction is the direction along the long side of the adhesive skin patch and the cross machine direction is the direction along the short side, wherein the tensile load is measured using JIS L 1096, and wherein the support layer comprises a woven fabric having a warp density of 80 to 120 yarns/inch (2.54 cm) and a weft density of 80 to 120 yarns/inch (2.54 cm), and using yarns having a total fineness of 40 dtex or more and 85 dtex or less, wherein a moisture permeability (g/m$^2$·24h) of the adhesive skin patch is 1,000 g/m$^2$·24h or more, wherein the moisture permeability is measured according to condition B of JIS Z 0208, at a temperature of 40°C and a relative humidity of 90%.

[12] The support layer for an adhesive skin patch described in [12], which is a woven fabric using a false twisted yarn having a total fineness of 40 dtex or more and 85 dtex or less, and a crimp recovery (CR) of 15 to 40%.

Effects of the Invention

The adhesive skin patch of the present invention can adequately and sufficiently suppress elongation of the skin, so that it is possible to sufficiently reduce the external force applied to the skin having scars due to external wounds or surgery, and is excellent in followability and adhesiveness to the skin, etc., whereby it can be adhered to the skin for a long term. Therefore, according to the adhesive skin patch of the present invention, it is possible to promote the healing of the wounds such as external wounds, wound site after surgery, etc., and treatment of the scars, and effectively prevent the formation of the scar or the keloid.

In addition, while the adhesive skin patch of the present invention is largely affected by the physical characteristics possessed by the support layer, the support layer for an adhesive skin patch of the present invention is a material having high elongation suitable for obtaining the above-mentioned adhesive skin patch.

BRIEF DESCRIPTION OF THE DRAWINGS

Fig. 1 is a drawing showing the shape of an adhesive skin patch which is an embodiment of the present invention.
Fig. 2 is a drawing showing the shape of an adhesive skin patch which is an embodiment of the present invention.

EMBODIMENT TO CARRY OUT THE INVENTION

In the following, the present invention is explained in detail.

<Adhesive skin patch>

The adhesive skin patch of the present invention (in the following, it is also referred to as "the said adhesive skin patch") has a support layer, and a pressure-sensitive adhesive layer provided on one surface of the support layer. Further, the said adhesive skin patch may have a release liner and/or a carrier sheet, if necessary. In addition, one or more other layers may be interposed between the carrier sheet and the support layer, between the support layer and the pressure-sensitive adhesive layer, and/or between the pressure-sensitive adhesive layer and the release liner. The support layer, the pressure-sensitive adhesive layer, the release liner, the carrier sheet, and the other layers are mentioned later. The direction along the long side of the adhesive skin patch is defined as the machine direction, and the direction along the short side as the cross machine direction. For example, in the adhesive skin patch having a rectangular shape, the direction along the long side is made the machine direction and the direction along the short side is made the cross machine direction, and in the case of a square, the direction of one side is made the machine direction and the other is made the cross machine direction. The same definitions apply when the adhesive skin patch is circular or oval. In the case of a roll shape, the longer direction is made the machine direction and the shorter direction is made the cross machine direction when it is cut.

The said adhesive skin patch has a 1% tensile load in the machine direction and the cross machine direction of 13 N/25 mm or less, and a 10% tensile load of the same of 18 N/25 mm or more and 95 N/25 mm or less. By having such physical characteristics, the said adhesive skin patch can appropriately suppress elongation of the skin in accordance with the stress which tends to stretch the skin, is also excellent in the ability to follow the skin, and can be adhered for a long time. That is, the adhesive skin patch of the present invention sufficiently follows to the skin by elongation under weak stress, while against strong stress, it firmly fixes to the same to have the effects of healing the wound, treating scars, or preventing formation of scars or keloids.

The above-mentioned 1% tensile load is 13 N/25 mm or less, preferably 11 N/25 mm or less, more preferably 10 N/25 mm or less, and further preferably 8 N/25 mm or less. If the above-mentioned 1% tensile load exceeds 15 N/25 mm, it suppresses a little elongation of the skin, so that there is a possibility that followability to the skin of the adhesive skin patch becomes insufficient. If the followability is insufficient, a feeling of discomfort during the adhesion is increased. The lower limit of the 1% tensile load in the preferred range is not particularly present, and is generally 0.01 N/25 mm or more.

The 5% tensile load is preferably 5 N/25 mm or more and 50 N/25 mm or less, more preferably 8 N/25 mm or more and 50 N/25 mm or less, and further preferably 8 N/25 mm or more and 45 N/25 mm or less. If the 5% tensile load is in the above-mentioned numerical range, it is difficultly deformed by a moderate external force, etc., whereby the suppressing effect of elongation of the skin is sufficient. In addition, it can suppress elongation of the skin, and followability to the skin of the adhesive skin patch in the state of adhering the said adhesive skin patch is also excellent so that a feeling of discomfort or skin irritation is difficultly caused during the adhesion.

The above-mentioned 10% tensile load is 18 N/25 mm or more and 95 N/25 mm or less, preferably 20 N/25 mm or more and 85/25 mm or less, more preferably 25 N/25 mm or more and 80 N/25 mm or less, and further preferably 30 N/25 mm or more and 80 N/25 mm or less. If the above-mentioned 10% tensile load is less than 18 N/25 mm, the suppressing effect of the adhesive skin patch against a strong external force, etc., is insufficient, and there is a fear that the skin is greatly elongated. Further, if the above-mentioned 10% tensile load exceeds 95 N/25 mm, elongation of the skin can be suppressed but in the state of adhering the adhesive skin patch, followability of the adhesive skin patch to the skin is markedly lowered, and a feeling of discomfort occurs during the adhesion so that normal operation may be hardly carried out, adhesion may be deteriorated, or skin irritation may occur, in some cases. Measurement of the tensile load in the present invention is carried out by subjecting to the tensile test according to JIS K 7113, and measurements at the 1%, 5% and 10% tensile loads are performed.

The said adhesive skin patch preferably has elongation recovery rates at loads of 9N and 18N in at least one of a machine direction and a cross machine direction of 85% or more. The said adhesive skin patch can suitably suppress elongation of the skin in accordance with the stress which makes the skin to be stretched, and in addition, after the stress applied to the skin disappears, the elongation recovery which can recover to the original length is desirable to be 85% or more. The elongation recovery rate is preferably 85% or more, and more preferably 90% or more. Even if the said adhesive skin patch is once extended due to the stress applied to the skin, it can be restored to a length close to the original length without becoming the stretched out state, so that the effect of suppressing elongation of the wound site can be maintained over a long time.

In the said adhesive skin patch, the bending resistance (mm) thereof according to the cantilever method is preferably 5 mm or more and 40 mm or less. When the said adhesive skin patch has characteristics that the bending resistance (mm) according to the cantilever method is 5 mm or more and 40 mm or less in addition to the characteristics of the above-mentioned tensile load, elongation of the skin can be suitably suppressed, external forces applied to the skin having scars due to external wounds or surgery can be sufficiently lowered, followability to the skin thereof is also excellent, and long term adhesion can be realized. The bending resistance (mm) according to the cantilever method in the said adhesive skin patch is preferably 5 mm or more and 40 mm or less, more preferably 8 mm or more and 25 mm or less, and further preferably 10 mm or more and 20 mm or less. In the adhesive skin patch, if the bending resistance (mm) according to the above-mentioned cantilever method is within the above-mentioned numerical range, followability to the skin and the suppressing effect of elongation of the skin are sufficient.

In the said adhesive skin patch, a product of the bending resistance (mm) according to the cantilever method and the apparent density (g/cm$^3$) is preferably 2 or more and 30 or less. The present inventors have found that, in the adhesive skin patch, the value of the product of the above-mentioned bending resistance and the apparent density has deep relation with the suppressing effect of elongation of the skin when the adhesive skin patch is adhered to the skin and long term adhesion, particularly with long term adhesion. In the said adhesive skin patch, the product of the above-mentioned bending resistance and the apparent density is preferably 2 or more and 30 or less, more preferably 3 or more and 25 or less, further preferably 6 or more and 22 or less, and particularly preferably 8 or more and 15 or less. If the product of the above-mentioned bending resistance and the apparent density is within the above-mentioned numerical range, the resulting patch is excellent in followability to the skin and long term adhesion. Here, "the bending resistance (mm) according to the cantilever method" can be measured in accordance with JIS L 1096.

In the said adhesive skin patch, the moisture permeability is 1,000 (g/m$^2$·24h) or more. When the moisture permeability of the said adhesive skin patch is 1,000 (g/m$^2$·24h) or more, there is little steaming when it is adhered to the skin, and skin irritation or itching during adhesion are unlikely to occur, so that long term adhesion becomes possible. The moisture permeability of the said adhesive skin patch is more preferably 2,000 (g/m$^2$·24h) or more, further preferably 3,000 (g/m$^2$·24h) or more, particularly preferably 5,000 (g/m$^2$·24h) or more, and most preferably 9,000 (g/m$^2$·24h) or more. The moisture permeability is preferably as high as possible while there is no particular upper limit of the preferred moisture permeability, and it is usually 30,000 (g/m$^2$·24h) or less. In the present invention, measurement of the moisture permeability is carried out according to Condition B of JIS Z0208 at a temperature of 40°C and a relative humidity of 90%. That is, one surface side of the adhesive skin patch was controlled at a temperature of 40°C and a relative humidity of 90%, a moisture absorbent such as calcium chloride, etc., was placed at the other surface side and the moisture passed through the adhesive skin patch was absorbed thereto, and an amount of weight change of the moisture absorbent was calculated in terms of 1 m$^2$ for 24 hours.

When the adhesive skin patch has the respective tensile loads, the elongation recovery rate at the time of loading, the bending resistance according to the cantilever method, the product of the bending resistance according to the cantilever method and the apparent density, and the moisture permeability within the above-mentioned defined ranges, it can suppress elongation of the skin as well as it is excellent in long term adhesion. That is, it can effectively prevent from deforming the skin suffered from scars by the external wound or surgery by a mechanical action such as an external force and extension of scar tissue, etc., so that it is useful as a material for healing the wounds, for treating scars, for preventing formation of scars and keloids. In addition, the said adhesive skin patch is also excellent in followability to the skin. Further, the said adhesive skin patch is also excellent in moisture permeability, so that it difficultly causes steaming when it is adhered to the skin, whereby it is possible to adhere the patch for a long time. To be able to adhere it for a long time, it is influenced by having excellent followability to the skin, sufficient adhesive property, and less feeling of discomfort during adhesion, skin irritation and itching at the time of adhesion, etc.

A thickness of the said adhesive skin patch is not particularly limited, and from the viewpoint of easiness of handling, it is 6 μm or more and 6 mm or less, preferably 50 μm or more and 2 mm or less, more preferably 60 μm or more and 1 mm or less, and further preferably 80 μm or more and 0.5 mm or less.

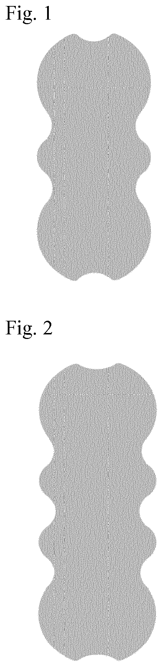

The shape of the said adhesive skin patch is not particularly limited as long as it has the above-mentioned support layer and the pressure-sensitive adhesive layer, and it can be made a free shape depending on the purpose including, for example, a quadrangle such as a rectangle, a square and a rhombus, etc., a polygon, an ellipse, a sheet state shape in which these shapes are optionally combined, a tape state in which it is continuously formed in a specific direction and a shape of a rolled state, etc. For the adhesive skin patch having a corner portion in the periphery, R may be optionally provided in the peripheral corner portion. Further, it may be formed in a three-dimensional shape in accordance with the part to be adhered, or a cut or a slit may be provided. When the concave-convex portions are provided in a machine direction or a cross machine direction and many curves are provided as the shapes having one or more concave portions or convex portions at the side portion sandwiched between both ends of the machine direction or the cross machine direction, then the stress at the time of stretching the skin is dispersed whereby there are advantages that the patch is difficultly peeled off and adhesion thereof is improved, etc. For example, it is preferred to make it a dumbbell shape (a shape like a cut plane in a machine direction of a dumbbell; see Figs. 1 and 2) or a corrugated shape (an edge of the adhesive skin patch is cut into a corrugated shape). In particular, as shown in Figs. 1 and 2, by making such a shape that one or more concave portions or convex portions are formed on the side portion sandwiched between both ends of the machine direction and the cross machine direction such as a shape in which arcuate notches are formed in the upper and lower tip portions of a dumbbell shape, it is possible to make it difficult to peel off against both the machine direction and the cross machine direction. The size of the said adhesive skin patch can be also freely selected depending on the purpose, and when it is adhered on the wound worn to the skin for the purpose of preventing from formation of scars or keloids, the patch is required to have a size which can sufficiently cover the wound. For example, as an adhesive skin patch to be used for caesarean section wound, it is preferred to have a size of the length of the wound (in the range of 5 cm to 15 cm in most cases) or more, such as a width of 3 cm to 8 cm and a length of 5 cm to 25 cm or so.

When the said adhesive skin patch is used for a medical purpose such as prevention of scars or keloids, etc., or adhering to a skin suffered from wound, etc., it may be subjected to a sterilization treatment to prevent from bacterial infection or virus infection, etc. As a method of the above-mentioned sterilization treatment, a method which has generally and widely been used for an adhesive skin patch may be used and, for example, γ ray sterilization method, electron beam sterilization method, high pressure steam sterilization method, ethylene oxide gas sterilization method, etc., may be used.

The said adhesive skin patch is preferably a material in which a medicine necessary for the affected part such as a wound, etc., can be coated thereon and administered to the part If so, it is not necessary to replace the adhesive skin patch by peeling off at every time of administering the medicine to the affected part, so that QOL of the patient can be improved, and one adhesive skin patch can be continuously used for a long time. The medicine to be coated onto the adhesive skin patch is not particularly limited, and a medicine which can improve the effects of the present invention is preferably used. For example, there may be mentioned an antibacterial agent, an anti-inflammatory analgesic, a steroid, an anesthetic, an antifungal agent, a vitamin preparation, etc. These medicines exert their effects on the whole body or local portion by percutaneous absorption, or exert their effect locally at the adhered site.

The said adhesive skin patch is preferably to block irradiation of ultraviolet rays to the wound area from the viewpoints of healing of wounds, treatment of scars, prevention of scars or keloids, etc. The ultraviolet transmittance of the said adhesive skin patch in the range of the wavelength of 280 to 400 nm is preferably 20% or less, further preferably 15% or less, and particularly preferably 10% or less.

In the preferred embodiment of the present invention, the above-mentioned medicine may contain a medicine which suppresses formation of scars or keloids. Such a medicine may contain an anti-allergic agent, a steroid, a TGF-β signal inhibitor, etc. It has been known that an allergic reaction is participated in generation or progression of scars or keloids, itching, pain, etc. Also, adrenocortical steroids have various functions, and in the skin, there are the effect of suppressing inflammation and the effect of atrophying a tissue, etc. TGF-β plays a very important role in maintaining homeostasis in living bodies, and it has been known that TGF-β signal abnormality causes various diseases such as scleroderma, skin sclerosis and various fibrosis, etc. It has been considered as a cause common to these diseases that excessive tissue fibrillization is induced by excessive TGF-β signal.

In the following, the support layer, the pressure-sensitive adhesive layer, the release liner, the carrier sheet, and other layers constituting the said adhesive skin patch will be described in detail.

[Support layer]

The support layer (in the following, it is also referred to as "the said support layer") of the adhesive skin patch of the present invention is not particularly limited as long as it can make a 1% tensile load in the machine direction and the cross machine direction of 13 N/25 mm or less and a 10% tensile load of the same of 18 N/25 mm or more and 95 N/25 mm or less when it is made an adhesive skin patch, and, for example, those made of the following materials may be mentioned.

As the material of the said support layer, it may be selected from

a polyester such as a polyethylene terephthalate (PET), a polybutylene terephthalate, etc.;
a polyamide such as Nylon (Nylon 6, Nylon 66), etc.;
a polyolefin such as a polypropylene, a polyethylene, a low density polyethylene (Low Density Polyethylene; LDPE), a high density polyethylene (High Density Polyethylene; HDPE), etc.;
an olefin-based copolymer such as an ethylene-vinyl acetate copolymer (EVA), an ethylene-ethyl acrylate copolymer (EEA), an ethylene-methyl acrylate copolymer (EMA), an ethylene-methyl methacrylate copolymer (EMMA), an ethylene-acrylic acid copolymer (EAA), etc.;
a polyvinyl alcohol (PVA);
polychlorides such as a polyvinyl chloride, a polyvinylidene chloride, etc.;
pulps using wood, etc., as a starting material;
silicones;
a polyurethane, etc., or a composite material thereof. Also, as for the material which has a low moisture permeability when it is used alone, it may be used by making porous by adding calcium carbonate, etc., or by processing by drilling, etc.

As the material of the said support layer, among these, a polyester, a polyamide, a polyolefin and a polyurethane are preferred, and above all, a polyethylene terephthalate (PET), Nylon, a polyethylene and pulp are more preferred, and a polyethylene terephthalate (PET), Nylon and a low density polyethylene (LDPE) are further preferred. Also, as the composite material, a composite material of a polyethylene terephthalate (PET) and pulp is preferred.

In addition, the said support layer contains a woven fabric comprising the above-mentioned materials, or may contain a nonwoven fabric, a knitted fabric, a film, a foamed body, etc. Among these, the said support layer preferably contains a woven fabric from the viewpoint that the said adhesive skin patch has sufficient moisture permeability, and the viewpoint that a medicine can be coated onto the adhesive skin patch at the time of adhesion and the viewpoint that the adhesive skin patch is elongated (the tensile load of 1% is small) following a minute movement at the time of adhesion but suppresses any movement which affects on the formation of scars or keloids (the tensile load of 10% is large). In the PET film, the tensile load of 1% is large, and it does not follow a minute movement at the time of the adhesion so that a feeling of discomfort during the adhesion is felt or the patch becomes easy to peel off in many cases. In the nonwoven fabric, although it follows a minute movement at the time of the adhesion, it does not suppress a movement which affects on the formation of scars or keloids in many cases (the tensile load of 10% is small).

Also, the said support layer may comprise a material in which a woven fabric, a nonwoven fabric, a knitted fabric, a film, a foamed body, etc., comprising the above-mentioned materials are further processed. For example, it may be coated by a polyurethane resin, an acrylic resin, etc., or may be laminated with a polyurethane film, etc. By providing a layer having flexibility between the support layer and the pressure-sensitive adhesive layer by subjecting to coating or laminating, etc., it is possible to reduce the physical stimulation without hindering fine movement of the skin. Further, even when the adhesive skin patch is stretched due to an external force, etc., it can return to the original state without stretching and prevent infiltration of water, etc., at the time of the adhesion.

The said support layer which can be used may be mentioned a PET woven fabric to which a polyurethane coating has been applied, a PET woven fabric to which an acrylic coating has been applied, a PET woven fabric laminated with a polyurethane film, a PET woven fabric laminated with an ether-based polyurethane film, a nonwoven fabric comprising a composite material of PET and a pulp, an LDPE film, a Nylon woven fabric to which an acrylic coating has been applied, and a polyurethane foamed body.

The characteristics possessed by the said adhesive skin patch largely depend on the physical characteristics possessed by the said support layer. In particular, in order to appropriately and sufficiently exhibit the effect of suppressing elongation of the skin, the 1% tensile load in the machine direction and the cross machine direction of the said support layer is required to be 13 N/25 mm or less, and the 10% tensile load of the same 15 N/25 mm or more and 95 N/25 mm or less. By using the support layer having different compositions and characteristics in the machine direction and the cross machine direction, a support layer having different strengths in the machine direction and the cross machine direction can be obtained. When the said support layer has such physical strength, the physical strength of the obtainable adhesive skin patch is also appropriate, the patch is difficultly deformed by an external force or elongation of the scar tissue, etc., and elongation of the skin can be effectively suppressed. The tensile strength is not limited only to the kind of the support layer, and if the woven fabric is used, it can be easily adjusted to the 1% tensile load of 15 N/25 mm or less and the 10% tensile load of 15 N/25 mm or more and 95 N/25 mm or less.

The said support layer is a woven fabric, in particular, using a yarn having a total fineness of 40 dtex or more and 85 dtex or less, and having a warp density of 80 to 120 yarns/inch (2.54 cm) and a weft density of 80 to 120 yarns/inch (2.54 cm).

If the total fineness is smaller than the above-mentioned lower limit, it becomes thin when made it into a woven fabric, and it tends to be difficult to handle, while if the total fineness is larger than the above-mentioned upper limit, a woven fabric becomes too thick and when it is made an adhesive skin patch, followability to the skin tends to be worsened.

Also, if the weaving density is smaller than the above-mentioned lower limit, it tends to cause loss of the agent or loss of a feeling of stiffness, while if the weaving density is larger than the above-mentioned upper limit, the comfort of attachment tends to be poor.

Further, the yarn to be used in the said support layer is preferably a false twisted yarn having a crimp recovery (CR) of 25 to 60%. If the crimp recovery is smaller than the above-mentioned lower limit, the 1% tensile load tends to be large, while if the crimp recovery is larger than the above-mentioned upper limit, the 10% tensile load tends to be small. Also, the strength of the false twisted yarn is preferably 3 to 6 cN/dtex, and the elongation thereof is preferably 15 to 40%.

The said support layer is more preferably a plain weave fabric using a polyester false twisted yarn.

Also, the said support layer can be obtained by weaving in a conventional manner and subjecting it to post treatment such as heat setting, etc., depending on the purpose.

The 1% tensile load of the said support layer is 13 N/25 mm or less, preferably 11 N/25 mm or less, more preferably 10 N/25 mm or less, and further preferably 8 N/25 mm or less. If the above-mentioned 1% tensile load exceeds 13 N/25 mm, slight skin elongation is also suppressed, and there is a possibility that followability to the skin of the obtainable adhesive skin patch becomes insufficient. If the followability is insufficient, a feeling of discomfort during the adhesion becomes remarkable. There is no particular lower limit of the 1% tensile load in the preferred range, and it is usually 0.01 N/25 mm or more.

The 5% tensile load of the said support layer is preferably 5 N/25 mm or more and 50 N/25 mm or less, more preferably 8 N/25 mm or more and 50 N/25 mm or less, and further preferably 8 N/25 mm or more and 45 N/25 mm or less. If the above-mentioned 5% tensile load is within the above-mentioned numerical range, it is difficultly deformed by a moderate external force, etc., and the suppressing effect of elongation of the skin by the obtainable adhesive skin patch becomes sufficient. Also, it can suppress the elongation of the skin, as well as it is excellent in followability to the skin, and a feeling of discomfort during the adhesion or skin irritation is difficultly generated.

The 10% tensile load of the said support layer is 15 N/25 mm or more and 95 N/25 mm or less, preferably 20 N/25 mm or more and 80 N/25 mm or less, more preferably 25 N/25 mm or more and 80 N/25 mm or less, and further preferably 30 N/25 mm or more and 80 N/25 mm or less. If the above-mentioned 10% tensile load is less than 15 N/25 mm, the obtainable adhesive skin patch has an insufficient suppression effect against a strong external force, etc., and there is a fear that the skin is greatly elongated. Also, if the above-mentioned 10% tensile load exceeds 95 N/25 mm, elongation of the skin can be suppressed, but in the state where the adhesive skin patch is adhered, followability of the adhesive skin patch to the skin is markedly lowered, and a feeling of discomfort occurs during the adhesion whereby it may be difficult to perform normal operation or skin irritation may occur in some cases.

A breaking strength of the said support layer is preferably 18 N/25 mm or more, more preferably 50 N/25 mm or more and further preferably 100 N/25 mm or more.

To the said support layer may be added an additive usually used depending on necessity. The above-mentioned additives may be mentioned, for example, an ultraviolet absorber, an aging inhibitor, a filler, a pigment, a colorant, a flame retardant, an antistatic agents, etc. These additives may be used alone or in combination of two or more, and a content of these additives may be selected an optimum range depending on the kind of the additive, and in the support layer, it is usually 0.001 to 30% by mass, preferably 0.01 to 25% by mass, more preferably in the range of 0.1 to 20% by mass in many cases. The said support layer is preferably to block irradiation of ultraviolet rays to a wound site by adding a UV absorber to the support layer or the UV absorber is coated onto the support layer, etc., from the viewpoints of healing of wounds, treatment of scars, prevention of scars or keloids, etc. The ultraviolet transmittance of the support layer within the wavelength range of 280 to 400 nm is preferably 20% or less, further preferably 15% or less, and particularly preferably 10% or less.

The thickness of the said support layer is, from the viewpoints of sufficiently exhibiting the effect of suppressing elongation of the skin of the adhesive skin patch of the present invention as well as heightening handling property, 5 $\mu$m or more and 5 mm or less, preferably 20 $\mu$m or more and 3 mm or less, more preferably 40 $\mu$m or more and 1 mm or less, further preferably 50 $\mu$m or more and 0.5 mm or less, and particularly preferably 60 $\mu$m or more and 0.15 mm or less. If the thickness of the said support layer is within the above-mentioned numerical range, an effect of suppressing skin elongation in the adhesive skin patch can be easily obtained and there is no fear of breaking the patch by friction, etc., at the time of use. In addition, it is also excellent in handling property, and when it is adhered to the skin, a feeling of discomfort is during the adhesion to the skin is difficultly caused, in particular, it is excellent in followability to a curved surface portion of the skin so that it is difficultly peeled off from the skin.

To the said support layer may be provided micro pores for the purpose of applying an ointment, a cream, a gel, etc., containing a medicine from above the support layer during the adhesion. For example, by applying an ointment containing a steroid from above the support layer, inflammation or itching at the wound part can be suppressed without peeling off the adhesive skin patch. The size of the micro pores is not particularly limited as long as the ointment, the cream or the gel permeates into the wound part, and is preferably 0.01 to 30 mm$^2$.

[Pressure-sensitive adhesive layer]

The pressure-sensitive adhesive layer (in the following, it is also referred to as "the said pressure-sensitive adhesive layer") of the adhesive skin patch of the present invention is provided on one surface of the above-mentioned support layer. The said pressure-sensitive adhesive layer has a role of detachably fixing the adhesive skin patch of the present invention to the skin. The said pressure-sensitive adhesive layer contains at least one adhesive. In addition, it may optional components such as a softener, a tackifier, a pH adjuster, a medicinal ingredient, a filler, an antioxidant (an antioxidant, an antiseptic), a coloring agent, a perfume, etc, if necessary. In the following, the adhesive and the above-mentioned optional components contained in the said pressure-sensitive adhesive layer are described in detail.

The kind of the adhesive contained in the said pressure-sensitive adhesive layer is not particularly limited and may be mentioned, for example, a urethane-based adhesive, an acrylic-based adhesive, a rubber-based adhesive, a silicone-based adhesive, etc.

The urethane-based adhesive may be mentioned a material comprising a urethane resin obtained by reacting a polyol and a polyisocyanate compound. The polyol may be mentioned, for example, polyether polyol, polyester polyol, polycarbonate polyol, polycaprolactone polyol, etc. The polyisocyanate compound may be mentioned, for example, diphenylmethane diisocyanate, tolylene diisocyanate, hexamethylene diisocyanate, etc.

The NCO/OH molar ratio of the polyol and the polyisocyanate compound to be used in the above-mentioned reaction is preferably made 0.7/1.00 to 1.00/1.00 so that gelation in the reaction is suppressed and the adhesive strength is increased.

For the production of the urethane-based adhesive, for example, usual methods such as a bulk polymerization (solid reaction) method which reacts in a molten state, and a solution polymerization method, etc., can be used. The solvent to be used in the solution polymerization method may be specifically mentioned a ketone-based solvent such as methyl ethyl ketone, methyl isobutyl ketone, cyclohexanone, etc., an ester-based solvent such as ethyl acetate, butyl acetate, etc., an ether-based solvent such as dioxane, tetrahydrofuran, etc., a glycol ether-based solvent such as Cellosolve, Carbitol, etc., an acetate glycol ether-based solvent such as Cellosolve acetate, etc., an amide-based solvent such as dimethylacetamide, dimethylformamide, etc., an aromatic hydrocarbon-based solvent such as toluene, xylene, etc., an alcohol-based solvent such as methanol, ethanol, isopropanol, etc., and further a mixed solvent of these.

When the urethane-based adhesive is produced, a catalyst and an additive, etc., may be used, if necessary. The catalyst may be mentioned general urethanation catalysts including a nitrogen-containing compound such as triethylamine, triethylenediamine, etc., and an organometallic compound such as dibutyltin dilaurate, tin octylate, tin stearate, etc. The additive may be mentioned, for example, a UV absorber such as a substituted benzotriazole, etc., an antioxidant such as a phenol derivative, etc., and a hydrolysis inhibitor, etc.

In order to increase the cohesive force of the urethane-based adhesive and to avoid problems such as adhesive residue, etc., a chain extender may be used for the preparation of the above-mentioned urethane-based adhesive. Further, adhesive properties of the urethane-based adhesive can be changed by using a polyisocyanate curing agent in combination. Increasing the cohesive force by using the polyisocyanate curing agent in combination is effective for improving defects such as adhesive residue, etc. As the polyisocyanate to be used as the curing agent, the polyisocyanate used in the above-mentioned reaction can be also used, and a polyol adduct of isocyanate obtained by the reaction of these with a bifunctional or higher polyol is preferred, and, polymeric polyisocyanate, an isocyanurate modified product and a carbodiimide modified product are also preferred. More specifically, there may be mentioned Coronate L, Coronate HL, Coronate 3041, Coronate 2030, Coronate 2031, Coronate HX all available from Nippon Polyurethane Industry Co., Ltd., and Millionate MTL, Millionate MR, etc.

The urethane-based adhesive has high safety and is practically used as a polymer material for medical use. It becomes an adhesive having high moisture permeability, good adhesiveness to the skin, soft, good conformability and low irritative property. Therefore, it is suitable for adhesion to a human body and is suitable as an adhesive for an adhesive skin patch for medical use. In addition, it satisfies all of adhesiveness, safety, stability as a material, economy, etc.

The acrylic-based adhesive may be mentioned, for example, homopolymers of an acrylic acid ester or a methacrylic acid ester which has an alkyl group having 4 to 12 carbon atoms such as butyl acrylate, isononyl acrylate, 2-ethylhexyl acrylate, 2-hydroxyethyl acrylate, isooctyl acrylate, n-octyl acrylate, etc.; a copolymer comprising these (meth)acrylic acid esters as main component and one or more copolymerizable other monomers such as (meth)acrylic acid, vinyl acetate, vinylpyrrolidone, acrylamide, hydroxyethyl acrylate, hydroxypropyl acrylate, etc., as the other monomers, being copolymerized. The other monomers are generally used in the range of 2 to 50% by mass.

The acrylic-based adhesive can be obtained by using a peroxide such as benzoyl peroxide as an initiator and polymerizing the monomers under a nitrogen atmosphere in an organic solvent such as toluene, hexane, ethyl acetate, etc. The acrylic-based adhesive obtained by this method is called a solvent type acrylic-based adhesive. Also, an emulsion type acrylic-based adhesive obtained by emulsifying and dispersing monomers in water with an emulsifier and then polymerizing them may be also used.

In order to crosslink the acrylic-based adhesive, after the polymerization, the acrylic-based adhesive is crosslinked by using a crosslinking agent such as a polyvalent isocyanate-based crosslinking agent, a polyvalent epoxy resin-based crosslinking agent, a polyvalent metal-based crosslinking agent, etc. Specifically, for example, Tetrad-X (available from Mitsubishi Gas Chemical Company, Inc.), NACEM (available from Nihon Kagaku Sangyo Co., Ltd.), Coronate HL, Coronate L, Coronate EH (available from Nippon Polyurethane Industry Co., Ltd.), etc., are used. These crosslinking agents are usually added to the adhesive composition when the adhesive composition is prepared.

By imparting cohesiveness by crosslinking the acrylic-based adhesive, it can be made the constitution that suitable adhesive force to the surface of the skin is maintained and adhesive residue is little when the patch is eliminated from the skin. The crosslinking agent is generally used in the range of 0.01 to 5 parts by mass based on 100 parts by mass of the acrylic-based adhesive (the acrylic-based polymer components), preferably 0.05 to 2 parts by mass. That is, the amount of the crosslinking agent to be used is optionally selected from the above-mentioned range, taking into consideration of the adhesive properties, cohesiveness, bleeding property of the medicinal ingredient(s), etc. To the acrylic-based adhesive may be formulated a liquid component such as a softening agent, if necessary.

The rubber-based adhesive may be mentioned, for example, a material in which a tackifier such as a rosin-based resin, a terpene-based resin, a coumarone-indene resin, a petroleum-based resin, etc., is added to a rubbery elastomer such as natural rubber, styrene-isoprene-styrene block copolymer, polyisobutylene, polybutene, polyisoprene, and a mixture of two or more kinds thereof, etc. To the rubber-based adhesive may be added, if necessary, a softening agent such as a liquid polybutene, a liquid polyisobutylene, mineral oil, etc.; a filler such as titanium oxide, zinc oxide, etc.; an antioxidant such as butylhydroxytoluene, etc. Among these, from the viewpoint of tackiness, those comprising a styrene-isoprene-styrene block copolymer as a main base material is preferred.

The silicone-based adhesive may be mentioned, for example, a mixture of silicone rubber and a silicone resin or a partial condensate thereof. The silicone rubber may be mentioned, a linear polydiorganosiloxane with a high molecular weight having silicon functional groups such as a silanol group at the both ends, and the silicone resin may be mentioned, a polyorganosiloxane having a branched or network structure which contains a monofunctional siloxane unit and a tetrafunctional siloxane unit, and has a silicon functional group such as a silanol group or a methoxy group in the molecule. More specifically, such a silicone rubber may be mentioned, a long chain copolymer of polydimethylsiloxane, and a silicone resin may be mentioned an MQ resin (a silicone resin with a three-dimensional structure comprising M unit $((CH_3)_3SiO_{1/2})$ and Q unit $(SiO_2)$).

The composition ratio of the silicone rubber/silicone resin constituting the silicone-based adhesive is not particularly limited, and preferably 30:70 to 60:40, more preferably 35:65 to 45:55 (mass ratio). Particularly preferred composition ratio of the silicone rubber/silicone resin in the present invention may be mentioned 40/60 (w/w) (BIO-PSA4501, Dow Corning Corp.), 45/55 (w/w) (BIO-PSA4601, Dow Corning Corp.), etc.

The silicone-based adhesive is an adhesive having a pressure-sensitive adhesive property by a silicon functional group present in the molecule. The organic group bonded to the silicon atom may be mentioned various monovalent hydrocarbon group such as methyl, ethyl, vinyl, phenyl, etc., and the adhesive property can be controlled by selecting the kind of the substituent. In the silicone-based adhesive, the intermolecular distance of the polyorganosiloxane the intermolecular distance thereof is large, so that it is enriched in air permeability and moisture permeability.

The adhesive contained in the said pressure-sensitive adhesive layer may be used each adhesive singly or in combination of two or more. In addition, the form of these adhesives may be the adhesive alone or, for example, a hydrogel containing water, a form of an organogel containing an organic solvent, or a hydrocolloid adhesive which is made a form of a hydrocolloid to which a hydrophilic polymer compound is added. The pressure-sensitive adhesive layer may be formed as a single adhesive or may be formed by laminating a plurality of pressure-sensitive adhesive layers. In the case where a plurality of pressure-sensitive adhesive layers are laminated, it is possible to laminate a plurality of different kinds of adhesives or laminate adhesives with different sizes. For example, a hydrocolloid adhesive that is smaller than the support layer can be used like a part of a pad of a bandage.

Among these adhesives, an acrylic-based adhesive or a foamed acrylic-based adhesive is preferred in view of the moisture permeability, low irritative property, safety, suitable adhesiveness, etc., and a foamed acrylic-based adhesive is further preferred.

The said pressure-sensitive adhesive layer may contain a softening agent, a tackifier, a pH adjuster, a medicinal ingredient, a filler, an antioxidant (antioxidant, antiseptic), a coloring agent and a perfume as an optional component(s).

The softening agent lowers elasticity of the adhesive to impart flexibility and improves adhesiveness, so that it may be added depending on necessity. The softening agent may be mentioned, for example, mineral oils such as liquid paraffin, liquid isoparaffin and naphthenic oil, etc.; vegetable oils such as olive oil and olive squalene, etc.; animal oils such as lanolin, turtle oil, beeswax, etc.; synthetic oils such as fatty acid triglyceride and silicone oil, etc.

The tackifier may be added depending on necessity since it can make it difficult to cause cohesive failure while imparting appropriate flexibility to the adhesive. The tackifier may be mentioned, for example, natural rosin derivatives, coumarone-indene resins, terpene oligomers, aliphatic petroleum resins, alkyl-modified phenolic resins, polyterpene resins, gum rosin, rosin esters, oily phenolic resins, coumarone-indene resins, petroleum-hydrocarbon resins, etc.

The kind of the pH adjusting agent is not particularly limited as long as it does not impair the object of the present invention and it may be mentioned, for example, karaya gum, citric acid, phosphoric acid, sodium hydrogenphosphate, anhydrous sodium hydrogenphosphate, pectin, citric anhydride, alkali metal hydroxides, buffers of organic acids, etc. By using these pH adjusting agents, the pressure-sensitive adhesive layer can be adjusted to the pH of the normal skin, and adjusted to a pH in the range of 4.0 to 6.0.

The medicinal ingredient that can be contained in the pressure-sensitive adhesive layer is not particularly limited, and a material which can improve the effect of the present invention is preferred. Specific examples thereof may be mentioned the same agents as those exemplified as the medicine which can be administered from above the said adhesive skin patch.

The thickness of the said pressure-sensitive adhesive layer is not particularly limited, and is preferably 1 $\mu$m or more and 1 mm or less from the viewpoint of ensuring the fixing property to the skin and the balance with the thickness of the support layer, more preferably 10 $\mu$m or more and 0.5 mm or less, and further preferably 20 $\mu$m or more and 0.1 mm or less. The coating weight (the adhesive thickness) of the said pressure-sensitive adhesive layer at the time of drying is preferably 1 g/m$^2$ or more and 1,000 g/m$^2$ or less, more preferably 10 g/m$^2$ or more and 200 g/m$^2$ or less, and further preferably 20 g/m$^2$ or more and 100 g/m$^2$ or less. If the thickness of the said pressure-sensitive adhesive layer or the coating weight is the above-mentioned upper limit or more, there is a fear of lowering moisture permeability and handling property. On the other hand, if it is less than the above-mentioned lower limit, there is a fear that the fixing property to the skin cannot be ensured.

The adhesive strength of the adhesive skin patch in which the pressure-sensitive adhesive layer is provided on such a surface is such that the adhesive strength is neither too strong nor too weak, and it is 0.3 to 10 N/25 mm or so in the adhesive strength to the BA-SUS plate, preferably 0.5 to 4.0 N/25 mm. The above-mentioned adhesive strength to the BA-SUS plate is measured by adhering a tape test piece prepared by cutting to a length of 150 mm and a width of 25 mm to a BA-SUS plate under an atmosphere of a temperature of 23°C and 50% RH, and press-bonded with 2 kg of a rubber roller back and forth once at a speed of 600 mm/min. Within 1 minute, peeling force is measured under conditions of a peeling angle of 180 degrees and a peeling rate of 300 mm/min, and the adhesive strength of the test piece to the BA-SUS plate is determined. The measurement is carried out three times, and the average value thereof is set as the adhesive strength of the adhesive skin patch to the BA-SUS plate (Unit: N/25 mm).

The tackiness of the pressure-sensitive adhesive layer provided on the support layer is not excessively strong nor too weak, and it is about 0.4 to 10 (N/5 mm$\varphi$) or so in the probe tack, preferably 0.6 to 5.0 (N/5 mm$\varphi$) or so. The above-mentioned probe tack is measured by using a probe tack tester manufactured by Nichiban Co., Ltd., according to the probe tack test method described in JIS Z 0237 as reference. This measurement is carried out three times, and the average value thereof is defined to be the pair-probe tack (Unit=N/5 mm$\varphi$).

[Release liner]

The release liner (in the following, it is also referred to as "the said release liner") to be used in the adhesive skin patch of the present invention is provided, for the purpose of protecting the pressure-sensitive adhesive layer, on the surface of the said pressure-sensitive adhesive layer opposite to the surface on which the support layer is provided, in the state of peelable. As the said release liner, release paper, release film, etc., commonly used in the field of the adhesive skin patch can be used. For example, paper based materials such as high quality paper subjected to silicone release treatment and glassine paper, etc., and a polyester film, etc., can be used. In addition, although the thickness of the release liner is not particularly limited, it is usually 20 μm or more, preferably 40 μm or more, and the upper limit value is 500 μm or so. By providing one or two or more linear dividing portions of the release liner which divide the outer shape of the release liner at substantially the center portion thereof, even when one of the release liner is peeled off, the other release liner(s) remains thereon, whereby it becomes possible to perform an adhering work without touching with the adhesive surface and workability is improved. When the adhesive skin patch is made in the form of a roll, it is particularly effective for making it easy to peel off the peeled body and to improve handling property. Further, in order to facilitate peeling of two or more release liners from the adhesive, one of the release liner may be placed so as to cover the other or fold back.

[Carrier sheet]

The carrier sheet (in the following, it is also referred to as "the said carrier sheet") to be used in the adhesive skin patch of the present invention is provided in a releasable state on the side opposite to the side provided with the pressure-sensitive adhesive layer of the support layer, and plays a role of improving manufacturability and operability of the adhesive skin patch of the present invention by reinforcing the support layer. Also, the carrier sheet is desirably transparent or translucent in consideration of visibility that allows the attachment site to be confirmed at the time of attachment. Further, the carrier sheet preferably has a relatively high modulus of elasticity with respect to the support layer and has an elastic modulus of about 3 to 20 times the support layer. In addition, it is appropriate to carry out various treatments at the surface of the support layer on which the carrier sheet is to be laminated since it is necessary to laminate it while maintaining appropriate adhesiveness with the support layer. Examples of such a treatment may be mentioned corona treatment, plasma treatment, ultraviolet ray treatment, mat treatment, etc.

When the said carrier sheet is difficultly peeled off from the support layer, a cut may be formed in the vicinity of the central portion of the carrier sheet, or the carrier sheet may be made two sheets by providing a gap between the carrier sheets being opened the cut. Also, a tape or a film may be further laminated on the upper portion of the cut of the carrier sheet to provide a mouth portion as a grasping piece. The mouth portion may be a film, a nonwoven fabric, a woven fabric or a laminate thereof, or an adhesive tape and coloring thereof is also possible. The end portion of the carrier sheet may have a corrugated shape or in the state of a plurality of notches, and a material which is formed larger than the support layer may be used. These are effective for facilitating peeling of the carrier sheet and improving handling properties even when the adhesive skin patch is made in the form of a roll.

A material used as the said carrier sheet may be mentioned, for example, a polyolefin such as a polyethylene, a polypropylene, etc., a polyester such as a polyethylene terephthalate, etc., a polyamide such as Nylon, etc., a polyvinyl chloride, a polyvinylidene chloride, etc. Also, it may be not only these carrier sheets in a single body, but also a carrier sheet of a composite laminated with paper, a nonwoven fabric, a woven fabric, a knitted fabric, a metal foil, and for such a carrier sheet, a polyolefin and a polyester film are preferably used from the viewpoint of visibility or a cost, etc.

The thickness of the said carrier sheet is not particularly limited, and it is usually 20 μm or more, preferably 40 μm or more, and the upper limit value thereof is 500 μm or so.

In the adhesive skin patch of the present invention, one or more other layers may be interposed between the carrier sheet and the support layer, between the support layer and the pressure-sensitive adhesive layer, and/or between the pressure-sensitive adhesive layer and the release liner. For example, a primer layer, a pressure-sensitive adhesive layer, or a peeling agent layer may be provided to enhance adhesiveness and releasability, or a film, a nonwoven fabric, a woven fabric, or a laminate thereof may be interposed.

<Producing method of adhesive skin patch>

The method for producing the said adhesive skin patch is not particularly limited, and it can be produced according to a general method for producing an adhesive skin patch.

The pressure-sensitive adhesive layer can be formed, for example, by supplying an adhesive onto one of the upper surface of the support layer or the release liner by a method such as a coating method, an extrusion molding method, etc. Also, it can be formed by supplying an adhesive between the support layer and the release liner. Further, the support layer and the pressure-sensitive adhesive layer may be coextruded.

It is preferred that the method is a method of producing an adhesive skin patch including a process of coating an adhesive on an upper surface of a release liner layer to form a pressure-sensitive adhesive layer from the viewpoint of capable of producing an adhesive skin patch with stable quality easily. The coating method of the adhesive is not particularly limited such as spread coating, hot melt coating, emulsion coating, etc., and, in particular, in the case of obtaining a thin pressure-sensitive adhesive layer having a thickness of 50 μm or less, a spread coating method is preferably used. Specifically, while running a preliminarily formed release liner in one direction, an adhesive for forming a pressure-sensitive adhesive layer is applied to the upper surface thereof, and the solvent is dried and removed to form a pressure-sensitive adhesive layer. Then, a support layer is laminated so that the pressure-sensitive adhesive layer is opposed to the release liner, whereby an adhesive skin patch characterized by comprising a support layer, a pressure-sensitive adhesive layer and a release liner in this order can be obtained.

As the coating pattern of the said pressure-sensitive adhesive layer, the surface of the said support layer may be coated entirely, and it is also possible to partially coat the surface. When it is coated partially, any form such as lattice, net, granular, arabesque, etc., can be selected. In this manner, by partially providing the said pressure-sensitive adhesive layer on one side of the said support layer, air permeability, moisture permeability, etc., can be further improved, and stimulation at the time of peeling from the skin can be further reduced.

At the time of producing the said adhesive skin patch, the said support layer may be subjected to a surface treatment or a primer treatment for the purpose of improving adhesiveness between the said support layer and the said pressure-sensitive adhesive layer. The surface treatment of the said support layer may be employed, for example, any known treatment method such as embossing, sand matting, corona discharge treatment, plasma treatment, alkali treatment, etc. The primer treatment can be carried out by using any conventionally known primer which can be used in composition according to the present invention, for example, using a primer comprising a silane coupling agent, etc.

<Usage and application of adhesive skin patch>

As explained above, the said adhesive skin patch can sufficiently reduce the external force applied to the skin having scars due to external wounds or surgery since it can suppress elongation of the skin when it is adhered to the skin. Also, it is excellent in followability to the skin, moisture permeability, etc., so that it can be adhered for a long time. Therefore, according to the adhesive skin patch of the present invention, it is possible to promote healing of wounds and treatment of scars, and effectively prevent formation of scars and keloids. In particular, it is effective for prevention of formation of scars and keloids. It is suitably used for treatment of scars of the wound site due to surgical operations such as a caesarean section, a median sternotomy incision or a laparoscopic operation, or prevention of formation of scars and keloids. The direction (the machine direction and the cross machine direction) of the adhesive skin patch at the time of adhering can be decided according to the size of the wound. In particular, when the wound is linear, it is preferably so adhered that the 10% tensile load in the direction (the direction to which the external force is likely to be applied) perpendicular to the wound becomes high. Also, in the uses other than these, there is no particular limitation as long as it is the use that can sufficiently exhibit the effects of the present invention. For example, it can be adhered to an affected part suffered from eczema, burns, acne, pierced hole, BCG inoculation, etc.

In general, the wound healing process is one of the biological defense systems which can be divided into three stages of an inflammation stage, a proliferation stage and a tissue reconstruction stage from the time course, and mild wounds are naturally healed without any treatment. However, if mechanical distortion such as elongation is generated at the wound part, in particular, removal of necrotic tissue by macrophages by digestion or apoptosis of extra cells in the early proliferation stage is suppressed which leads to scarring of collagen fibers, and as a result, it can be presumed that reconstruction of the tissue is suppressed and the scar is enlarged whereby a keloid is formed. Therefore, the adhesive skin patch according to the present invention can be used for treating scars due to external wounds or surgery from the viewpoint of promoting wound healing, and on the other hand, in the sense of suppressing formation of scars and keloids, it can be used for prevention of formation of scars and keloids.

The application of the said adhesive skin patch is optionally selected depending on the purpose and, for example, when the object is to prevent formation of scars or keloids at the wound part, it is preferred to adhere the patch for 3 to 12 months continuously. When one sheet of the said adhesive skin patch is once adhered, then, it can be adhered continuously until it is peeled off. This term is usually about 3 days to 10 days. Thereafter, it is desired that a new adhesive skin patch is adhered, and when it is peeled off, then, a new adhesive skin patch is adhered, which procedure is carried out repeatedly.

The said adhesive skin patch may be covered by using a dressing material or a medical tape, etc., from above the said adhesive skin patch for the purpose of waterproofing and long-term adhesion, if necessary.

EXAMPLES

In the following, the present invention is explained in more detail by referring to Examples. Part(s) and % in the following Examples and Comparative examples represent part(s) by mass and % by mass, respectively, otherwise specifically mentioned.

<<Production of adhesive skin patch>>

1. Adhesive

As the acrylic adhesive, the acrylic adhesive 1 and the acrylic adhesive 2 were used. The acrylic adhesive 1 is an acrylic-based adhesive in which 0.3 part by mass of Coronate (Registered Trademark) HL (available from Nippon Polyurethane Industry Co., Ltd.) had been added as a crosslinking agent to 100 parts by mass of an acrylic acid alkyl ester copolymer comprising isononyl acrylate/2-methoxyethyl acrylate/acrylic acid (mass ratio: 68/30/2). The acrylic adhesive 2 is an acrylic-based adhesive in which 0.03 part by mass of TETRAD-X (Registered Trademark) (available from Mitsubishi Gas Chemical Company, Inc.) had been added as a crosslinking agent to 100 parts by mass of an acrylic acid alkyl ester copolymer comprising isononyl acrylate/acrylic acid (mass ratio: 96/4).

2. Support layer

As the support layer,
PET woven fabric,
PET woven fabric + PU film laminated product (a material in which a polyester woven fabric is laminated by a polyurethane film),
A material in which one surface of PET woven fabric is coated by a urethane-based resin,
PET film,
PP spunbond nonwoven fabric (polypropylene long fiber nonwoven fabric),
Polyurethane nonwoven fabric, and
PET/pulp nonwoven fabric (nonwoven fabric containing a polyester and pulp)
were used.

The above-mentioned PET woven fabric was prepared by the method shown below. Specifically, a plane knitted fabric (width: 135 cm, warp density: 102 yarns/inch (2.54 cm), weft density: 90 yarns/inch (2.54 cm)) was weaved by using a false twisted yarn (56 dtex/36 yarns, crimp recovery (CR): 36%, strength: 4.6 cN/dtex) obtained by false twisting polyethylene terephthalate fibers (available from KB SEIREN Co., Ltd., 84 dtex/36 yarns, containing 0.3% by mass of titanium oxide) at a torque of 178 turns/m, and the grey fabric was produced under the conditions shown in Table 1 to produce each PET woven fabric. The width, the weaving density, the thickness and the basis weight of each PET woven fabric are also shown in Table 1. Also, the 1%, 5% and 10% tensile load of each PET woven fabric are shown in Table 2. Measurement methods of the crimp recovery (CR) of the above-mentioned a false twisted yarn, and the size, characteristics, etc., of each PET woven fabric are as shown below.

<Measurement method of each characteristic of support layer>

(Tensile load)

Tensile tests were carried out according to JIS L 1096, respectively, and the 1%, 5% and 10% tensile loads were measured. The test pieces of each adhesive skin patch used were those in which a rectangle with a width of 25 mm and a length of 100 mm was cut for each of the cross machine direction (CD) and the machine direction (MD), respectively. Three sheets of test pieces were cut for each of the cross machine direction (CD) and the machine direction (MD) of each support layer, measurements were carried out on all three sheets and the average value of the three sheets was made the measured value of the tensile load in the respective CD direction and the MD direction. The measurement conditions are summarized as follows.
Testing machine: Autograph tensile testing machine

Test piece: width of 25 mm and length of 100 mm
Grip distance: 50 mm
Cross head moving speed (tensile speed): 300 mm/min
Repeated test number: n=3

(Weaving density)

Using a densitometer, the weave densities in each direction of warp and weft were measured and these were taken as the weaving density in that direction.

(Thickness)

A thickness of each of four corners of the test piece with 250 mm × 250 mm was measured after pressing for 10 seconds using a thickness measuring instrument (Model No. H, manufactured by PEACOCK) according to JIS L 1085, and the average value was taken as the measured value.

(Basis weight)

Three sheets of the test pieces with 250 mm $\times$ 250 mm were taken, the mass up to 10 mg was measured, and the average value was multiplied by 16. The average value of three was taken as the measured value.

(Crimp recovery (CR))

A small skein having a skein length of about 40 cm with 10 turns is prepared by applying thereto a load of 0.176 mN $\times$ total fineness, and boils at 100°C for 15 minutes. This sample was immersed in water for 2 minutes at a temperature of 20$\pm$2°C by applying thereto a load of 0.176 mN $\times$ 20 $\times$ total fineness and further a load of 8.82 mN $\times$ 20 $\times$ total fineness, then, the skein length was measured and the load of 8.82 mN $\times$ 20 $\times$ total fineness was immediately removed and after it was allowed to stand for 2 minutes, the skein length was again measured and the crimp recovery (%) was calculated by the following formula.

$$E = (a-b) \times 100/a$$

$$E = \text{Crimp recovery (\%)}$$

a=Skein length (mm) when a load of 8.82 mN $\times$ 20 $\times$ total fineness is further applied to the load of 0.176 mN $\times$ 20 $\times$ total fineness
b=Skein length (mm) when a load of 0.176 mN $\times$ 20 $\times$ total fineness is applied

[Table 1]

| | | Finishing process (heat set) | Width | Density (yarns/inch (2.54 cm)) | | Thickness | Basis weight |
|---|---|---|---|---|---|---|---|
| | | | cm | Warp | Weft | mm | g/m$^2$ |
| Example | 1 | None | 131 | 101 | 88 | 0.16 | 48 |
| | 2 | Done | 131 | 101 | 93 | 0.16 | 51 |
| | 3 | None | 136 | 95 | 87 | 0.13 | 46 |
| | 4 | None | 122 | 106 | 100 | 0.22 | 60 |
| | 5 | None | 131 | 101 | 88 | 0.17 | 71 |
| | 6 | Done | 131 | 101 | 93 | 0.18 | 61 |

[Table 2]

| | | 1% Tensile load (N/25 mm) | | 5% Tensile load (N/25 mm) | | 10% Tensile load (N/25 mm) | |
|---|---|---|---|---|---|---|---|
| | | MD | CD | MD | CD | MD | CD |
| Example | 1 | 8.6 | 9.3 | 28.7 | 30.0 | 56.0 | 56.3 |
| | 2 | 9.1 | 8.6 | 31.5 | 26.4 | 60.2 | 52.9 |
| | 3 | 9.8 | 9.6 | 43.7 | 33.7 | 72.1 | 57.6 |
| | 4 | 8.1 | 8.5 | 20.6 | 20.6 | 45.1 | 42.6 |
| | 5 | 8.3 | 8.2 | 25.0 | 18.3 | 54.6 | 37.6 |
| | 6 | 7.8 | 4.5 | 54.1 | 16.0 | 97.3 | 53.7 |

3. Preparation of adhesive skin patch

[Examples 7 to 12 and Comparative examples 1 to 6]

An ethyl acetate solution (the total solid content is about 40% by mass) of an acrylic adhesive was coated onto one surface of the release liner so as to have a thickness after drying of 40 g/m$^2$, then, dried to form a pressure-sensitive adhesive layer, and each support layer was laminated thereto to prepare an adhesive skin patch. The acrylic adhesive and the support layer used were shown in Table 3, respectively. The characteristics of the obtained adhesive skin patches were measured by the methods mentioned later and shown in Tables 4 to 8. Incidentally, "-" in Table 6 indicates that it is not implemented.

[Table 3]

| | | | Constitution | |
|---|---|---|---|---|
| | | | Support | Adhesive agent |
| Examples | 7 | PET woven fabric (Example 1) | Acrylic adhesive 1 |
| | 8 | PET woven fabric (Example 2) | Acrylic adhesive 1 |
| | 9 | PET woven fabric (Example 3) | Acrylic adhesive 1 |
| | 10 | PET woven fabric (Example 4) | Acrylic adhesive 1 |
| | 11 | PET woven fabric (Example 1) + polyurethane film; Support of Example 5 | Acrylic adhesive 1 |
| | 12 | PET woven fabric (Example 2) + urethane coating; Support of Example 6 | Acrylic adhesive 1 |
| Comparative examples | 1 | PET film | Acrylic adhesive 1 |
| | 2 | PET film | Acrylic adhesive 1 |
| | 3 | PET film | Acrylic adhesive 1 |
| | 4 | PP spunbond nonwoven fabric | Acrylic adhesive 1 |
| | 5 | Polyurethane nonwoven fabric | Acrylic adhesive 2 |
| | 6 | PET/pulp nonwoven fabric | Acrylic adhesive 1 |

<Measurement method of each characteristic of adhesive skin patch>

(Tensile load)

With regard to the adhesive skin patches of Examples and Comparative examples, a tensile test was carried out according to JIS K 7113, respectively, and measurements of 1%, 5% and 10% tensile loads were carried out. The test pieces of each adhesive skin patch used were those in which a rectangle with a width of 25 mm and a length of 100 mm was cut for each of the cross machine direction (CD) and the machine direction (MD), respectively. Cloth adhesive tape <LS> No. 123 available from Nichiban Co., Ltd. were adhered to both ends so that the distance between the chucks became 50 mm, and test pieces for measuring the tensile strength were prepared and attached to the cross head of a Tensilon type tensile tester at a length of specimen between grips of the above-mentioned 50 mm. The upper cross head was raised at a speed of 300 mm/min, and the above-mentioned test piece was pulled. The condition setting the measurement of elongation was made "a starting point of elongation is an initial load point, an initial load value is 3.0 N, and loose correction is none", and the loads reached to 1%, 5% and 10% elongation were made 1%, 5% and 10% tensile loads, respectively. The unit of the tensile load was expressed by Newton (N)/25 mm. Three test pieces were cut for each of the cross machine direction (CD) and the machine direction (MD) of each adhesive skin patch, the above-mentioned measurements were carried out on all three sheets. The average value of the three sheets was made the measured value of the tensile load in the respective CD direction and the MD direction. The measurement conditions are summarized as follows.

Testing machine: Tensilon type tensile tester

Test piece: width of 25 mm and length of 100 mm
Grip distance: 50 mm
Cross head moving speed (tensile speed): 300 mm/min
Repeated test number: n=3
Measurement atmosphere: 23°C and 50% RH

(Bending resistance)

This is by the Cantilever test according to JIS L 1096. The test pieces of each adhesive skin patch used were those in which a rectangle with a width of 20 mm and a length of 150 mm was cut for each of the cross machine direction (CD) and the machine direction (MD), respectively. The test piece was placed on a horizontal base having a slope of 45° and the surface of which is smooth so that the short side of the sample was on the base line of the scale, and gently slid in the direction of the slope by an appropriate method. When the test piece was collapsed within 20 seconds, the position of the edge at the side of the horizontal base was read by the scale. The bending resistance was indicated by the length (mm) of the test piece moved, and the average value of the three sheets was made the measured value of the bending resistance. All measurements were carried out with the surface of the support layer surface of each adhesive skin patch down.

(Apparent density)

A value obtained by dividing the basis weight (g/m$^2$) of each adhesive skin patch by its thickness ($\mu$m) was calculated to be the apparent density (g/cm$^3$).

(Moisture permeability)

The moisture permeability was measured according to the condition B of JIS Z 0208, at a temperature of 40°C and a relative humidity of 90%. That is, one surface side of each adhesive skin patch was controlled at a temperature of 40°C and a relative humidity of 90%, about 15 g of a moisture absorbent (calcium chloride) was placed at the other surface side and the moisture passed through each adhesive skin patch was absorbed thereto, and an amount of weight change of the moisture absorbent was calculated in terms of 1 m$^2$ for 24 hours. The average value of the three sheets of the test pieces was made the moisture permeability.

(Dosage possibility from over adhesive skin patch)

It was evaluated whether or not it could be medicated from above the adhesive skin patch.
Two sheets of the adhesive skin patches cut into a width of 25 mm and a length of 100 mm were adhered to the lower part of the navel, and one each of the patch on the side not coated with the adhesive of the support layer was coated an ointment (Rinderon VG ointment 0.12%: available from Shionogi & Co., Ltd.) or 0.5 g of a cream (Rinderon VG cream 0.12%: available from Shionogi & Co., Ltd.). Thereafter, the adhesive skin patch was peeled off, and it was visually confirmed whether the ointment was applied on the skin or not, and evaluated as A when it was coated, and D when it was not coated. The test was carried out with three persons, and the evaluation of two or more persons was evaluated in this test.

(Elongation recovery rate)

The test pieces of each adhesive skin patch used were those in which a rectangle with a width of 25 mm and a length of 100 mm was cut for each of the cross machine direction (CD) and the machine direction (MD), respectively. Cloth adhesive tape <LS> No. 123 available from Nichiban Co., Ltd. were adhered to both ends so that the distance between the chucks became 50 mm, and test pieces for measuring the tensile strength were prepared and attached to the cross head of a Tensilon type tensile tester at a length of specimen between grips of the above-mentioned 50 mm (L0) by marking. The upper cross head was raised at a speed of 300 mm/min, and a length (L1) of the tape when a load of 9N (or 18N) was applied to the above-mentioned test piece was measured. Thereafter, a length (L2) between the chucks when the load began to be applied at the time of applying a load of 9 N (or 18 N) was measured after the displacement was returned to zero (removing the load), and it was obtained by the following formula.

$$\text{Elongation recovery rate (\%)} = \{(L1-L2)/(L1-L0)\} \times 100$$

Three sheets of test pieces were cut for each of the cross machine direction (CD) and the machine direction (MD) of each adhesive skin patch, measurements were carried out on all three sheets. The average value of the three sheets was made the measured value of the elongation recovery rate in the respective CD direction and the MD direction, respectively. The measurement conditions are summarized as follows.

Testing machine: Tensilon type tensile tester

Test piece: width of 25 mm and length of 80 mm
Between chucks: 50 mm
Cross head moving speed (tensile speed): 300 mm/min
Repeated test number: n=3
Measurement atmosphere: 23°C and 50% RH

[Table 4]

| | | 1% Tensile load (N/25 mm) | | 5% Tensile load (N/25 mm) | | 10% Tensile load (N/25 mm) | |
|---|---|---|---|---|---|---|---|
| | | MD | CD | MD | CD | MD | CD |
| Example | 7 | 4.0 | 4.0 | 12.8 | 14.0 | 43.7 | 45.7 |
| | 8 | 4.8 | 3.7 | 23.9 | 9.4 | 61.5 | 31.2 |
| | 9 | 7.2 | 5.8 | 44.5 | 32.4 | 77.4 | 66.8 |
| | 10 | 3.9 | 3.6 | 7.6 | 7.5 | 21.1 | 22.7 |
| | 11 | 5.1 | 4.0 | 26.0 | 10.3 | 62.7 | 28.3 |
| | 12 | 6.9 | 4.4 | 48.2 | 15.3 | 92.0 | 50.2 |
| Comparative example | 1 | 19.5 | 20.4 | 64.3 | 59.4 | 63.1 | 58.7 |
| | 2 | 27.0 | 27.5 | 109.8 | 100.6 | 110.2 | 101.1 |
| | 3 | 31.0 | 36.8 | 136.9 | 130.0 | 138.7 | 134.3 |
| | 4 | 14.1 | 8.2 | 20.5 | 11.0 | 20.5 | 11.6 |
| | 5 | 3.1 | 3.1 | 3.1 | 3.1 | 4.2 | 3.9 |
| | 6 | 13.6 | 8.6 | 21.0 | 14.2 | 33.2 | 29.6 |

[Table 5]

| | | Characteristics | | | | | |
|---|---|---|---|---|---|---|---|
| | | Bending resistance (mm) | Total thickness ($\mu$m) | Apparent density (g/cm$^3$) | Bending resistance $\times$ Apparent density | Moisture permeability (g/m$^2 \cdot$ 24h) | Dosage possibility from over patch |
| Example | 7 | 17 | 189 | 0.503 | 9 | 14530 | A |
| | 8 | 17 | 169 | 0.562 | 10 | 13920 | A |
| | 9 | 17 | 126 | 0.694 | 12 | 13380 | A |
| | 10 | 18 | 220 | 0.455 | 8 | 15140 | A |
| | 11 | 10 | 207 | 0.543 | 5 | 1050 | D |
| | 12 | 15 | 174 | 0.761 | 11 | 1150 | D |
| Comparative example | 1 | 30 | 66 | 1.136 | 34 | 68 | D |
| | 2 | 47 | 80 | 1.188 | 56 | 68 | D |
| | 3 | 34 | 91 | 1.209 | 41 | 68 | D |
| | 4 | 24 | 215 | 0.384 | 9 | 4480 | A |
| | 5 | 38 | 333 | 0.411 | 16 | 12760 | A |
| | 6 | 48 | 100 | 0.825 | 40 | 1120 | A |

Table 6]

| | | Elongation recovery rate (%) at 9N load | | Elongation recovery rate (%) at 18N load | |
|---|---|---|---|---|---|
| | | MD | CD | MD | CD |
| Example | 7 | 92.8 | 92.3 | 94.6 | 94.4 |
| | 8 | 90.4 | 94.7 | 91.0 | 94.2 |
| | 9 | 92.1 | 88.2 | 89.3 | 89.9 |
| | 10 | 94.3 | 94.5 | 92.1 | 88.1 |
| | 11 | 88.4 | 91.6 | 92.2 | 96.6 |
| | 12 | 88.5 | 89.6 | 90.8 | 91.3 |
| Comparative example | 1 | - | - | 92.9 | 92.0 |
| | 2 | - | - | - | - |
| | 3 | - | - | - | - |
| | 4 | 92.3 | 86.8 | 81.5 | 96.7 |
| | 5 | 89.7 | 90.7 | 90.9 | 90.2 |
| | 6 | 83.6 | 92.3 | 94.1 | 90.7 |

<Method of evaluation of adhesive skin patch (Practical evaluation on human skin)>

Each adhesive skin patch was adhered to each part mentioned below of a human skin and evaluated by the following method. For the evaluation at each part, three sheets of the test pieces in which each adhesive skin patch was cut with a size of 50 mm in each of the cross machine direction (CD) and the machine direction (MD) were used.

**EP 3 298 996 B1**

(Below navel)

A square with a size of 50 mm square (4 sides of 50 mm × 50 mm) was marked at the lower part of the navel with a felt tip pen. The vertical (up and down) direction of the body was made machine, and the horizontal (right and left) direction was made cross machine (the chest and shoulder portions were also similarly carried out). A person was stopped at the state in which both hands were put on one's hip and the hip was bent backwards (a state of bending backwards), and a length of the machine side marked on that occasion was measured (an action of elongating the skin). Also, a person was stopped at the state sitting deeply in the chair, and a length of the machine side marked on that occasion was measured (an action of contracting the skin). These measured values were used as measured values at the time of not adhered (blank). Next, the test piece of each adhesive skin patch was adhered to the marked portion, the same operation was carried out, and the length of the tape machine was measured with a measuring tape, which was made the measured value at the time of adhering. Similar measurements were carried out on three sheets of the test pieces of each adhesive skin patch. A difference between each of the measured value at the time of not adhered and the measured value at the time of adhered, and the initial value (50 mm) was calculated on how much the elongated or contracted length at the time of adhering was suppressed at the time of adhesion, which was calculated as a suppression rate (%) and evaluated according to the following evaluation criteria. Evaluation results only in the machine direction are shown in Table 7. The results shown in Table 7 are average values for the above-mentioned three sheets of the test pieces. Here, the suppression rate is 100% means that the elongation of the skin was completely suppressed by adhering the adhesive skin patch.

(Chest)

A square with a size of 50 mm square (4 sides of 50 mm × 50 mm) was marked at the portion of the chest moved frequently (the center of the chest) with a felt tip pen. Both hands were made a fist, and a person was stopped at the state that one's chest out so as to push the chest forward (an action of elongating the skin), and these lengths at the sides of machine and cross machine marked at that time were measured, which were made the measured values at the time of not adhered (blank). Next, the test piece of each adhesive skin patch was adhered to the marked portion, the same operation was carried out, and the lengths of the tape at the machine and cross machine sides were measured with a measuring tape, which were made the measured values at the time of adhering. Similar measurements were carried out on three sheets of each adhesive skin patch in each of the cross machine direction (CD) and the machine direction (MD). Calculation of the suppressing rate was carried out in the same manner as in the case of the navel portion and evaluated according to the following evaluation criteria. The results are shown in Table 7.

(Evaluation criteria)

About an act of stretching the skin

A: Suppressing rate of 100% to 90%
B: Suppressing rate of 89% to 80%
C: Suppressing rate of 79% to 70%
D: Suppressing rate of 69% or less

[Table 7]

| | | Result of practical evaluation (%) on human skin | | | | | | Total evaluation |
| --- | --- | --- | --- | --- | --- | --- | --- | --- |
| | | Below navel | | Chest (elongation) | | | | |
| | | Elongation (action of bending backwards) | | Machine | | Cross machine | | Suppression of elongation |
| | | MD | CD | MD | CD | MD | CD | |
| Example | 7 | 100 | 100 | 98 | 92 | 94 | 100 | A |
| | 8 | 100 | 100 | 100 | 88 | 100 | 100 | A |
| | 9 | 100 | 100 | 100 | 100 | 100 | 100 | A |
| | 10 | 96 | 100 | 88 | 92 | 80 | 92 | B |
| | 11 | 100 | 100 | 100 | 100 | 100 | 100 | A |
| | 12 | 100 | 100 | 100 | 100 | 100 | 100 | A |
| Comparative example | 1 | 100 | 100 | 100 | 100 | 100 | 100 | A |
| | 2 | 100 | 100 | 100 | 100 | 100 | 100 | A |
| | 3 | 100 | 100 | 100 | 100 | 100 | 100 | A |
| | 4 | 95 | 96 | 100 | 92 | 63 | 92 | D |
| | 5 | 85 | 85 | 92 | 83 | 40 | 90 | D |
| | 6 | 100 | 100 | 100 | 100 | 100 | 100 | A |

<Method of evaluation of adhesive skin patch (Evaluation of 5-day continuous adhesion on human skin)>

Each adhesive skin patch was adhered to the abdomen of a human skin and evaluated according to the method described below. For evaluation of each part, the test pieces of each adhesive skin patch cut into each size of 50 mm square in the cross machine direction (CD) and the machine direction (MD) were adhered one by one to the abdomen for 5 consecutive days, and adhesiveness, itching during adhesion, a feeling of discomfort during adhesion, pain at the time of peeling, and skin irritation after peeling at 1 hour and 24 hours were evaluated.

(Adhesiveness)

The area ratio adhered was calculated, and the average point was used as the evaluation result.

(Itching)
100: No itching
60: There is a little itching but no problem
0: There is itching and it is a problem
(Feeling of discomfort)
100: There is no feeling of discomfort
60: There is a slight discomfort but no problem
0: There is a feeling of discomfort and it is a problem
(Skin irritation after peeling at 1 hour and 24 hours)
100: No skin irritation
60: There is a little skin irritation but no problem
0: There is skin irritation and it is a problem

(Evaluation)

The results of adhesiveness, itching during adhesion, a feeling of discomfort during adhesion, pain during adhesion and skin irritation after peeling for 1 hour and 24 hours were scored based on the above-mentioned criteria, and the rating was as follows.

A: 100 to 90

B: 89 to 80

C: 79 to 70

D: 69 or less

[Table 8]

| | | Evaluation | | | | |
|---|---|---|---|---|---|---|
| | | Adhesiveness (after adhesion of 5 days) | Feeling of discomfort (during adhesion of 5 days) | Itching (during adhesion of 5 days) | Skin irritation (1 hour after peeling) | Skin irritation (24 hours after peeling) |
| Example | 7 | A | A | A | A | A |
| | 8 | A | A | A | A | A |
| | 9 | A | A | A | A | A |
| | 10 | A | A | A | A | A |
| | 11 | A | A | B | C | A |
| | 12 | B | B | B | C | A |
| Comparative example | 1 | D | D | D | D | D |
| | 2 | D | D | D | D | D |
| | 3 | D | D | D | D | D |
| | 4 | A | A | A | A | A |
| | 5 | A | A | A | A | A |
| | 6 | C | B | D | D | A |

<Evaluation>

As shown in the above-mentioned Tables 4 to 8, it could be understood that the adhesive skin patches of Examples sufficiently suppresses elongation of the skin. Also, the adhesive skin patches of Examples had a high adhesion rate to the skin after 5 days of adhesion, and there was almost no itching or a feeling of discomfort during adhesion. Further, skin irritation after peeling was small. Thus, it could be understood that the adhesive skin patches of Examples were excellent in the suppressing effect of elongation of the skin and excellent in followability to the skin, whereby it was possible to adhere for a long time. Specifically, the adhesive skin patches of Examples elongated following a minute movement at the time of adhesion (1% tensile load is small) and suppressed the movement affecting formation of scars and keloids (10% tensile load is large). To the contrary, the adhesive skin patches of Comparative examples were superior in the characteristic evaluation in Table 8, but it could be understood that there were materials in which the suppressing effect of elongation of the skin shown in Table 7 was insufficient, etc., whereby it was impossible to achieve both the suppressing effect of elongation of the skin and followability to the skin. In particular, in the PET films of Comparative examples 1 to 3, the 1% tensile loads were large, and these often did not follow minute movements at the time of adhesion, and often felt a feeling of discomfort during adhesion or easily peeled off. In the nonwoven fabrics of Comparative examples 4 to 6, the nonwoven fabric followed the minute movement at the time of adhesion, but often did not suppress the movement affecting formation of scars and keloids scars (the 10% tensile load is small). It could be understood that the 10% tensile strength affected the suppression of elongation, the 1% tensile strength, the bending resistance, and the product of the bending resistance and the apparent density affected adhesion and a feeling of discomfort, and the moisture permeability affected itching and skin irritation during adhesion. Incidentally, there is a material in which the difference in the measured values of the tensile loads of the support layer itself and the adhesive skin patch using the same support layer, and this is considered mainly by change in physical properties of the support layer by the tension and heat applied to the support layer at the time of coating the pressure-sensitive adhesive, and

22

heating at the time of aging the adhesive, etc.

INDUSTRIAL APPLICABILITY

According to the adhesive skin patch for covering wound or for protecting post-surgical wound of the present invention, elongation of the skin can be appropriately suppressed depending on the stress to be applied, so that the external force applied to the skin having scars due to external wounds or surgery can be sufficiently reduced, and also it is excellent in followability to the skin, moisture permeability, etc. Therefore, it is possible to adhere for a long term. Accordingly, the adhesive skin patch for covering wound or for protecting post-surgical wound of the present invention is suitable for healing wounds, for treating scars or for preventing formation of scars or keloids.

**Claims**

1. An adhesive skin patch for covering wound or for protecting post-surgical wound which comprises a support layer and a pressure-sensitive adhesive layer provided on one side of the support layer, wherein a 1% tensile load in a machine direction and a cross machine direction of the adhesive skin patch is 13 N/25 mm or less and a 10% tensile load in the machine direction and the cross machine direction is 18 N/25 mm or more and 95 N/25 mm or less, wherein the machine direction is the direction along the long side of the adhesive skin patch and the cross machine direction is the direction along the short side, wherein the tensile load is measured using JIS K7113, and wherein the support layer comprises a woven fabric having a warp density of 80 to 120 yarns/inch (2.54 cm) and a weft density of 80 to 120 yarns/inch (2.54 cm), and using yarns having a total fineness of 40 dtex or more and 85 dtex or less, wherein a moisture permeability ($g/m^2 \cdot 24h$) of the adhesive skin patch is 1,000 $g/m^2 \cdot 24h$ or more, wherein the moisture permeability is measured according to condition B of JIS Z 0208, at a temperature of 40°C and a relative humidity of 90%.

2. The adhesive skin patch according to Claim 1, wherein a bending resistance of the adhesive skin patch is 5 mm or more and 40 mm or less, and wherein the bending resistance is measured using JIS L1096.

3. The adhesive skin patch according to Claim 1 or 2, wherein a 5% tensile load in the machine direction and the cross machine direction of the adhesive skin patch is 5 N/25 mm or more and 50 N/25 mm or less.

4. The adhesive skin patch according to any one of Claims 1 to 3, wherein a product of the bending resistance (mm) according to a cantilever method and an apparent density ($g/cm^3$) is 2 or more and 30 or less.

5. The adhesive skin patch according to any one of Claims 1 to 4, wherein an elongation recovery rate of at least one of the machine direction and the cross machine direction of the adhesive skin patch at a load of 9 N and 18 N is 85% or more, and wherein the elongation recovery rate is measured by a method as set out in the description.

6. The adhesive skin patch according to any one of Claims 1 to 5, for use in healing wounds, treating scars or in preventing formation of scars and keloids.

7. The adhesive skin patch for use according to Claim 6, for use in healing wounds of a wound by surgery, treating scars or preventing formation of scars and keloids.

8. The adhesive skin patch for use according to Claim 7, wherein the surgery is a caesarean section, a median sternotomy incision or a laparoscopic operation.

9. The adhesive skin patch according to claim 1, for use in healing wounds of a wound by surgery, treating scars or preventing formation of scars and keloids, wherein
a 5% tensile load in a machine direction and a cross machine direction of the adhesive skin patch is 5 N/25 mm or more and 50 N/25 mm or less, and the pressure-sensitive adhesive layer contains a foamed acrylic-based adhesive.

10. The adhesive skin patch according to claim 1, for use in healing wounds of a wound by surgery, treating scars or preventing formation of scars and keloids, wherein
a 5% tensile load in a machine direction and a cross machine direction of the adhesive skin patch is 5 N/25 mm or more and 50 N/25 mm or less, a bending resistance (mm) of the adhesive skin patch is 8 mm or more and 25 mm or less, and a shape of which has one or more concave portions or convex portions on a side portion sandwiched between both ends in a machine direction or a cross machine direction.

11. A support layer for an adhesive skin patch which is used for a support layer of an adhesive skin patch for covering wound or for protecting post-surgical wound, wherein a 1% tensile load in a machine direction and a cross machine direction of the support layer is 13 N/25 mm or less and a 10% tensile load in the machine direction and the cross machine direction is 15 N/25 mm or more and 95 N/25 mm or less, wherein the machine direction is the direction along the long side of the adhesive skin patch and the cross machine direction is the direction along the short side, wherein the tensile load is measured using JIS L 1096, and
wherein the support layer comprises a woven fabric having a warp density of 80 to 120 yarns/inch (2.54 cm) and a weft density of 80 to 120 yarns/inch (2.54 cm), and using yarns having a total fineness of 40 dtex or more and 85 dtex or less, wherein a moisture permeability (g/m$^2$·24h) of the adhesive skin patch is 1,000 g/m$^2$·24h or more, wherein the moisture permeability is measured according to condition B of JIS Z 0208, at a temperature of 40°C and a relative humidity of 90%.

12. The support layer for an adhesive skin patch according to Claim 11, which is a woven fabric using a false twisted yarn having a total fineness of 40 dtex or more and 85 dtex or less, and having a crimp recovery (CR) of 15 to 40%.

**Patentansprüche**

1. Hautklebepflaster zum Bedecken von Wunden oder zum Schutz von postoperativen Wunden, das eine Trägerschicht und eine druckempfindliche Klebeschicht umfasst, die auf einer Seite der Trägerschicht angeordnet ist, wobei eine 1 %ige Zuglast in eine Maschinenrichtung und eine Maschinenquerrichtung des Hautklebepflasters 13 N/25 mm oder weniger beträgt und eine 10 %ige Zuglast in die Maschinenrichtung und die Maschinenquerrichtung 18 N/25 mm oder mehr und 95 N/25 mm oder weniger beträgt, wobei die Maschinenrichtung die Richtung entlang der Längsseite des Hautklebepflasters ist und die Maschinenquerrichtung die Richtung entlang der kurzen Seite ist, wobei die Zuglast unter Verwendung von JIS K7113 gemessen wird, und
wobei die Trägerschicht ein Gewebe mit einer Kettfadendichte von 80 bis 120 Fäden/Zoll (2,54 cm) und einer Schussfadendichte von 80 bis 120 Fäden/Zoll (2,54 cm) umfasst und Fäden mit einer Gesamtfeinheit von 40 dtex oder mehr und 85 dtex oder weniger verwendet werden, wobei eine Feuchtigkeitspermeabilität (g/m$^2$•24 h) des Hautklebepflasters 1.000 g/m$^2$•24 h oder mehr beträgt, wobei die Feuchtigkeitspermeabilität gemäß Bedingung B von JIS Z 0208 bei einer Temperatur von 40 °C und einer relativen Feuchte von 90 % gemessen wird.

2. Hautklebepflaster nach Anspruch 1, wobei eine Biegesteifigkeit des Hautklebepflasters 5 mm oder mehr und 40 mm oder weniger beträgt und wobei die Biegesteifigkeit unter Verwendung von JIS L1096 gemessen wird.

3. Hautklebepflaster nach Anspruch 1 oder 2, wobei eine 5 %ige Zuglast in Maschinenrichtung und Maschinenquerrichtung des Hautklebepflasters 5 N/25 mm oder mehr und 50 N/25 mm oder weniger beträgt.

4. Hautklebepflaster nach einem der Ansprüche 1 bis 3, wobei ein Produkt der Biegesteifigkeit (mm) gemäß einem Kragträgerverfahren und einer Rohdichte (g/cm$^3$) 2 oder mehr und 30 oder weniger beträgt.

5. Hautklebepflaster nach einem der Ansprüche 1 bis 4, wobei eine Dehnungsrückformungsrate von zumindest einer aus der Maschinenrichtung und der Maschinenquerrichtung des Hautklebepflasters bei einer Belastung von 9 N und 18 N 85 % oder mehr beträgt und wobei die Dehnungsrückformungsrate durch ein Verfahren wie in der Beschreibung dargelegt gemessen wird.

6. Hautklebepflaster nach einem der Ansprüche 1 bis 5 zur Verwendung in der Wundheilung, bei der Behandlung von Narben oder beim Verhindern der Bildung von Narben und Keloiden.

7. Hautklebepflaster zur Verwendung nach Anspruch 6 zur Verwendung in der Wundheilung einer postoperativen Wunde, bei der Behandlung von Narben oder beim Verhindern der Bildung von Narben und Keloiden.

8. Hautklebepflaster zur Verwendung nach Anspruch 7, wobei der chirurgische Eingriff ein Kaiserschnitt, eine Sternotomie oder ein laparoskopischer Eingriff ist.

9. Hautklebepflaster nach Anspruch 1 zur Verwendung in der Wundheilung einer postoperativen Wunde, bei der Behandlung von Narben oder beim Verhindern der Bildung von Narben und Keloiden, wobei

eine 5 %ige Zuglast in eine Maschinenrichtung und eine Maschinenquerrichtung des Hautklebepflasters 5 N/25 mm oder mehr und 50 N/25 mm oder weniger beträgt und wobei die druckempfindliche Klebeschicht einen geschäumten Klebstoff auf Acrylbasis enthält.

10. Hautklebepflaster nach Anspruch 1 zur Verwendung in der Wundheilung einer postoperativen Wunde, bei der Behandlung von Narben oder beim Verhindern der Bildung von Narben und Keloiden, wobei
eine 5 %ige Zuglast in eine Maschinenrichtung und eine Maschinenquerrichtung des Hautklebepflasters 5 N/25 mm oder mehr und 50 N/25 mm oder weniger beträgt, eine Biegesteifigkeit (mm) des Hautklebepflasters 8 mm oder mehr und 25 mm oder weniger beträgt und dessen Form einen oder mehrere konkave Abschnitte auf einem Seitenabschnitt aufweist, der sandwichartig zwischen beiden Enden in einer Maschinenrichtung oder einer Maschinenquerrichtung angeordnet ist.

11. Trägerschicht für ein Hautklebepflaster, die für eine Trägerschicht eines Hautklebepflasters zum Bedecken einer Wunde oder zum Schützen einer postoperativen Wunde verwendet wird, wobei eine 1 %ige Zuglast in einer Maschinenrichtung und einer Maschinenquerrichtung der Trägerschicht 13 N/25 mm oder weniger beträgt und eine 10 %ige Zuglast in der Maschinenrichtung und der Maschinenquerrichtung 15 N/25 mm oder mehr und 95 N/25 mm oder weniger beträgt, wobei die Maschinenrichtung die Richtung entlang der Längsseite des Hautklebepflasters ist und die Maschinenquerrichtung die Richtung entlang der kurzen Seite ist, wobei die Zuglast unter Verwendung von JIS L 1096 gemessen wird,
wobei die Trägerschicht ein Gewebe mit einer Kettfadendichte von 80 bis 120 Fäden/Zoll (2,54 cm) und einer Schussfadendichte von 80 bis 120 Fäden/Zoll (2,54 cm) umfasst, und Fäden mit einer Gesamtfeinheit von 40 dtex oder mehr und 85 dtex oder weniger verwendet werden, wobei eine Feuchtigkeitspermeabilität (g/m$^2$•24 h) des Hautklebepflasters 1.000 g/m$^2$•24 h oder mehr beträgt, wobei die Feuchtigkeitspermeabilität gemäß Bedingung B von JIS Z 0208 bei einer Temperatur von 40 °C und einer relativen Feuchte von 90 % gemessen wird.

12. Trägerschicht für ein Hautklebepflaster nach Anspruch 11, die ein Gewebe ist, in dem falsch verdrehte Fäden mit einer Gesamtfeinheit von 40 dtex oder mehr und 85 dtex oder weniger und mit einer Crimp-Rückformung (CR) von 15 bis 40 % verarbeitet sind.

## Revendications

1. Timbre transdermique adhésif pour recouvrir une plaie ou protéger une plaie postopératoire, qui comprend une couche de support et une couche adhésive sensible à la pression disposée sur un côté de la couche de support, dans lequel une charge de traction de 1 % dans la direction de la machine et la direction transversale à la machine du timbre transdermique adhésif est inférieure ou égale à 13 N/25 mm et une charge de traction de 10 % dans la direction de la machine et la direction transversale à la machine est supérieure ou égale à 18 N/25 mm et inférieure ou égale à 95 N/25 mm, dans lequel la direction de la machine est la direction le long du côté long du timbre transdermique adhésif et la direction transversale à la machine est la direction le long du côté court, dans lequel la charge de traction est mesurée à l'aide de JIS K7113, et
dans lequel la couche de support comprend un tissu tissé présentant une densité de fils de chaîne de 80 à 120 fils/pouce (2,54 cm) et une densité de fils de trame de 80 à 120 fils/pouce (2,54 cm), et utilisant des fils présentant une finesse totale de 40 dtex ou plus et de 85 dtex ou moins, dans lequel une perméabilité à l'humidité (g/m$^2$.24h) du timbre transdermique adhésif est de 1 000 g/m$^2$.24h ou plus, dans lequel la perméabilité à l'humidité est mesurée selon la condition B de JIS Z 0208, à une température de 40°C et une humidité relative de 90 %.

2. Timbre transdermique adhésif selon la revendication 1, dans lequel une résistance à la flexion du timbre transdermique adhésif est de 5 mm ou plus et de 40 mm ou moins, et dans lequel la résistance à la flexion est mesurée en utilisant JIS L1096.

3. Timbre transdermique adhésif selon la revendication 1 ou 2, dans lequel une charge de traction de 5 % dans la direction de la machine et la direction transversale à la machine du timbre transdermique adhésif est de 5 N/25 mm ou plus et de 50 N/25 mm ou moins.

4. Timbre transdermique adhésif selon l'une quelconque des revendications 1 à 3, dans lequel un produit de la résistance à la flexion (mm) selon un procédé en porte-à-faux et d'une densité apparente (g/cm$^3$) est de 2 ou plus et de 30 ou moins.

**5.** Timbre transdermique adhésif selon l'une quelconque des revendications 1 à 4, dans lequel un taux de récupération d'allongement d'au moins l'une de la direction de la machine et de la direction transversale à la machine du timbre transdermique adhésif à une charge de 9 N et 18 N est de 85 % ou plus, et
dans lequel le taux de récupération d'allongement est mesuré par un procédé tel que décrit dans la description.

**6.** Timbre transdermique adhésif selon l'une quelconque des revendications 1 à 5, à utiliser dans la cicatrisation de plaies, le traitement de cicatrices ou dans la prévention de la formation de cicatrices et de chéloïdes.

**7.** Timbre transdermique adhésif à utiliser selon la revendication 6, à utiliser dans la cicatrisation de plaies d'une plaie par chirurgie, le traitement de cicatrices ou la prévention de la formation de cicatrices et de chéloïdes.

**8.** Timbre transdermique adhésif à utiliser selon la revendication 7, dans lequel la chirurgie est une césarienne, une incision de sternotomie médiane ou une opération laparoscopique.

**9.** Timbre transdermique adhésif selon la revendication 1, à utiliser dans la cicatrisation d'une plaie par chirurgie, le traitement de cicatrices ou la prévention de la formation de cicatrices et de chéloïdes, dans lequel une charge de traction de 5 % dans la direction de la machine et la direction transversale à la machine croisée du timbre transdermique adhésif est de 5 N/25 mm ou plus et de 50 N/25 mm ou moins, et la couche adhésive sensible à la pression contient un adhésif à base d'acrylique en mousse.

**10.** Timbre transdermique adhésif selon la revendication 1, à utiliser dans la cicatrisation d'une plaie par chirurgie, le traitement de cicatrices ou la prévention de la formation de cicatrices et de chéloïdes, dans lequel une charge de traction de 5 % dans la direction de la machine et la direction transversale à la machine du timbre transdermique adhésif est de 5 N/25 mm ou plus et de 50 N/25 mm ou moins, une résistance à la flexion (mm) du timbre transdermique adhésif est de 8 mm ou plus et de 25 mm ou moins, et une forme de celui-ci présente une ou plusieurs parties concaves ou parties convexes sur une partie latérale enserrée entre les deux extrémités dans la direction de la machine ou la direction transversale à la machine.

**11.** Couche de support pour un timbre transdermique adhésif qui est utilisée pour une couche de support d'un timbre transdermique adhésif pour couvrir une plaie ou pour protéger une plaie postopératoire, dans laquelle une charge de traction de 1 % dans la direction de la machine et la direction transversale à la machine de la couche de support est inférieure ou égale à 13 N/25 mm et une charge de traction de 10 % dans la direction de la machine et la direction transversale à la machine est supérieure ou égale à 15 N/25 mm et inférieure ou égale à 95 N/25 mm, dans lequel la direction de la machine est la direction le long du côté long du timbre transdermique adhésif et la direction transversale à la machine est la direction le long du côté court, dans laquelle la charge de traction est mesurée à l'aide de JIS L 1096, et
dans lequel la couche de support comprend un tissu tissé présentant une densité de fils de chaîne de 80 à 120 fils/pouce (2,54 cm) et une densité de fils de trame de 80 à 120 fils/pouce (2,54 cm), et utilisant des fils présentant une finesse totale de 40 dtex ou plus et de 85 dtex ou moins, dans lequel une perméabilité à l'humidité (g/m$^2$.24h) du timbre transdermique adhésif est de 1 000 g/m$^2$.24h ou plus, dans lequel la perméabilité à l'humidité est mesurée selon la condition B de JIS Z 0208, à une température de 40 °C et une humidité relative de 90 %.

**12.** Couche de support pour un timbre transdermique adhésif selon la revendication 11, qui est un tissu tissé utilisant un fil à fausse torsion présentant une finesse totale de 40 dtex ou plus et de 85 dtex ou moins, et présentant une récupération de frisure (CR) de 15 à 40 %.

Fig. 1

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012135582 A
- JP 2009545382 A
- JP HEI07079827 B
- JP 2010281004 A